# EUROPÄISCHE PATENTSCHRIFT

(11) **EP 3 996 766 B1**
(45) Veröffentlichungstag und Bekanntmachung des Hinweises auf die Patenterteilung: **02.08.2023**
(21) Anmeldenummer: 20796502.1
(22) Anmeldetag: 15.10.2020
(51) Int. Cl.: A61M 1/00, A61B 5/24, A61B 17/00, A61B 5/0537, A61B 5/262, A61B 5/06, A61B 5/383, A61B 5/00, A61B 5/388, A61B 90/30, A61B 34/20

(54) **BIPOLARER MAPPINGSAUGER**
BIPOLAR MAPPING SUCTION DEVICE
DISPOSITIF D'ASPIRATION À MAPPAGE BIPOLAIRE

(30) Priorität: 18.10.2019 DE 102019216119
(43) Veröffentlichungstag der Anmeldung: 18.05.2022
(73) Patentinhaber: inomed Medizintechnik GmbH, 79312 Emmendingen (DE)
(72) Erfinder: MATTMUELLER, Rudi, 79312 Emmendingen (DE)
(74) Vertreter: Isarpatent
(86) Internationale Anmeldenummer: PCT/EP2020/078984
(87) Internationale Veröffentlichungsnummer: WO 2021/074265

(56) Entgegenhaltungen:
- DE-A1-102010 019 796
- US-A1- 2017 042 528
- SEIDEL KATHLEEN ET AL: "Continuous dynamic mapping to avoid accidental injury of the facial nerve during surgery for large vestibular schwannomas", NEUROSURGICAL REVIEW, GRUYTER, BERLIN, DE, Bd. 43, Nr. 1, 26. Oktober 2018 (2018-10-26), Seiten 241-248, XP037016983, ISSN: 0344-5607, DOI: 10.1007/S10143-018-1044-Z [gefunden am 2018-10-26]

## Beschreibung

### GEBIET DER ERFINDUNG

Die vorliegende Erfindung betrifft einen bipolaren Mappingsauger, für chirurgische Zwecke und ein System zum Absaugen von Flüssigkeiten und Gewebe sowie zum Überwachen von Nervengewebe.

### TECHNISCHER HINTERGRUND

Chirurgische Eingriffe am menschlichen Körper, beispielsweise Tumorresektionen, bringen je nach Operationsgebiet ein hohes Risiko für ungewollte Nervenschädigungen mit sich. Vor allem bei Operationen in der Nähe von wichtigen funktionellen Zentren und Nerven ist es besonders wichtig, Nervengewebe nicht zu verletzen, um motorische, sensible und autonome, aber auch psychische Einschränkungen des Patienten zu vermeiden. Intraoperatives Neuromonitoring ist eine etablierte Methode für die Überwachung von Nervengewebe und kann beispielsweise bei einer Tumorresektion (z. B. im Gehirn) angewendet werden. Besonders risikoreich sind Eingriffe, bei denen der Tumor unmittelbar an Nervengewebe wie einem Nerven oder einer Nervenbahn anliegt. Um dieses Nervengewebe bei chirurgischen Eingriffen weitestgehend zu schonen und dessen Verlauf zu identifizieren, wird das intraoperative Neuromonitoring in der klinischen Praxis eingesetzt. Hierbei wird das zu untersuchende Gewebe stimuliert und geprüft, ob eine Reizantwort detektiert wird. Dazu legt ein Chirurg oder ein anderer Anwender des OP-Personals während des Eingriffs eine Stimulationssonde bzw. Stimulationselektrode an das Gewebe und stimuliert dieses, beispielsweise elektrisch. Gleichzeitig wird in der Regel über Ableitelektroden, beispielsweise Nadelelektroden oder Oberflächenelektroden, am Patienten ein evoziertes Potential, beispielhaft ein Elektromyogramm (EMG) abgeleitet und dem Chirurgen das Antwortsignal auf einem Nervenmonitor zur Interpretation präsentiert. Somit kann bestimmt werden, ob es sich um gesundes Gewebe handelt oder nicht. Zur Identifikation des Nervenverlaufs tastet der Chirurg das Operationsgebiet mit der Stimulationssonde ab. Außerdem können basierend auf verwendeten Stimulationsintensitäten Abstände zu motorischen Bahnen abgeschätzt werden. Durch die Differenzierung zwischen gesundem und tumorösem Gewebe wird eine effektivere Tumorresektion ermöglicht.

Zudem muss während eines chirurgischen Eingriffs der Situs, unabhängig von der Lage eines Tumors, möglichst frei von (Körper-)Flüssigkeiten sein, um die Sicht des Chirurgen nicht zu beeinträchtigen. Für die freie Sicht des Chirurgen bzw. des Anwenders auf den Situs sollte dieser möglichst frei von Flüssigkeiten wie Blut oder anderen Körperflüssigkeiten sein. Dies ermöglicht es dem Chirurgen Gewebe, welches geschont werden muss, frühzeitig zu erkennen.

Beispielsweise bei Tumorresektionen erfolgt je nach Tumor und Operationsgebiet der Eingriff minimal-invasiv über einen kleinen Hautschnitt oder über natürlichen Körperöffnungen. Außerdem kann bei großen Tumoren eine "offene" Operation mit großem Hautschnitt notwendig sein. Die Auswahl der geeigneten Operationstechnik ist abhängig von der Lage und Ausdehnung des Tumors, wobei alle Methoden das Ziel einer möglichst ausgedehnten Tumorentfernung, ohne Beeinträchtigungen von Nervengewebe und dessen Funktion haben.

Ausgehend davon sind daher die Ziele, einerseits Tumorgewebe und Flüssigkeiten abzusaugen und andererseits gleichzeitig Nervengewebe kontinuierlich zu lokalisieren und dessen Funktion während des Eingriffs (z. B. Tumorresektion) zu überwachen, um Nervenschädigungen mit möglichen Folgen für den Patienten zu vermeiden. Wünschenswert ist ein Instrument, das das Gewebe kontinuierlichen stimuliert und dem Anwender eine Rückmeldung gibt, ob es sich um Gewebe handelt, welches entfernt werden muss, oder nicht. Gleichzeitig sollte Nervengewebe und dessen Verlauf identifiziert und geschont werden. (Körper-)Flüssigkeiten sollten zu jeder Zeit abgesaugt werden können, sodass der Anwender eine uneingeschränkte Sicht auf den trockenen Situs erhält.

Um beide Ziele zu erreichen, werden spezielle Sauger verwendet, die sowohl Flüssigkeiten wie beispielsweise Blut oder Spülmedium absaugen, als auch das Gewebe elektrisch stimulieren können. Bei monopolaren Saugern befindet sich nur ein Stimulationspol am Sauger selbst. Als zweiter Pol wird wenigstens eine zusätzliche Gegenelektrode (z. B. Nadelelektrode oder Oberflächenelektrode) angebracht. Die Platzierung von Nadelelektroden birgt generell die Gefahr von Blutungen, weshalb eine patientenschonendere Methode wünschenswert ist. Zusätzlich ist bei dieser Methode der Abstand zwischen den beiden Stimulationspolen groß und daher nur eine hochsensitive, jedoch keine selektive Messung möglich.

Dies ist ein Zustand, den es zu verbessern gilt.

### ZUSAMMENFASSUNG DER ERFINDUNG

Vor diesem Hintergrund liegt der vorliegenden Erfindung die Aufgabe zugrunde, das intraoperative Neuromonitoring mit der Resektion von Gewebe bzw. dem Entfernen von Flüssigkeiten aus dem Situs zu vereinen und zudem Nachteile des Stands der Technik zu beseitigen oder wenigstes zu lindern.

Erfindungsgemäß wird diese Aufgabe durch einen bipolaren Mappingsauger, für chirurgische Zwecke mit den Merkmalen des Patentanspruchs 1 und/oder ein System zum Absaugen von Flüssigkeiten und Gewebe sowie zum Überwachen von Nervengewebe mit den Merkmalen des weiteren unabhängigen Patentanspruch gelöst. Weiterbildungen und Ausgestaltungen sind Gegenstand der entsprechenden abhängigen Patentansprüche.

Das Sauginstrument und das System gemäß der vorliegenden Erfindung sind zu einer monopolaren oder bipolaren Stimulation von Gewebe jeglicher Art zur Differenzierung zwischen tumorösem und gesundem Gewebe und zur Lokalisierung von umliegendem Nervengewebe und dessen Verlauf sowie zu einem parallelen oder sequentiellen Absaugen von Gewebe und Flüssigkeiten wie beispielsweise Blut geeignet. Dadurch können Nervenschädigungen während eines chirurgischen Eingriffs vermieden werden.

Gemäß einem ersten Aspekt der vorliegenden Erfindung umfasst ein bipolarer Mappingsauger, für chirurgische Zwecke, ein Handstück, eine erste Schnittstelle, eine zweite Schnittstelle und eine Kanüleneinheit. Die erste Schnittstelle ist ausgebildet, zumindest eine Fluidverbindung mit einer externen Saugvorrichtung und zusätzlich oder alternativ Spülvorrichtung herzustellen. Die zweite Schnittstelle ist ausgebildet, eine bipolare elektrische Verbindung mit einer externen Stimulationseinrichtung herzustellen. Die Kanüleneinheit erstreckt sich von dem Handstück aus in eine axiale Richtung und ist an einem proximalen Ende der Kanüleneinheit mit dem Handstück mechanisch verbunden. Die Kanüleneinheit umfasst ein elektrisch leitfähiges äußeres Kanülenrohr, ein elektrisch leitfähiges inneres Kanülenrohr und eine Isolation. Das elektrisch leitfähige innere Kanülenrohr ist mit einem ersten Pol der bipolaren elektrischen Verbindung der zweiten Schnittstelle elektrisch verbunden. Das elektrisch leitfähige innere Kanülenrohr ist konzentrisch in dem äußeren Kanülenrohr angeordnet. Das elektrisch leitfähige innere Kanülenrohr ist mit der ersten Schnittstelle fluidleitend verbunden. Das elektrisch leitfähige äußere Kanülenrohr ist mit einem zweiten Pol der bipolaren elektrischen Verbindung der zweiten Schnittstelle elektrisch verbunden. Die Isolation ist konzentrisch zwischen dem äußeren Kanülenrohr und dem inneren Kanülenrohr angeordnet. Die Isolation ist ausgebildet, das äußere Kanülenrohr und das innere Kanülenrohr vollständig gegeneinander elektrisch zu isolieren.

Gemäß einem zweiten Aspekt der vorliegenden Erfindung umfasst ein System zum Absaugen von Flüssigkeiten und Gewebe und zusätzlich oder alternativ Spülen eines Situs sowie zum Überwachen von Nervengewebe das Sauginstrument gemäß dem ersten Aspekt der vorliegenden Erfindung, eine Saugvorrichtung und zusätzlich oder alternativ eine Spülvorrichtung, mindestens eine, vorzugsweise zwei Ableitelektroden sowie ein IOM-System (IOM: Intraoperatives Neuromonitoring). Die mindestens eine, vorzugsweise zwei Ableitelektroden sind an einem Patienten in der Nähe zu überwachenden Nervengewebes anbringbar. Das IOM-System umfasst eine Stimulationseinrichtung und eine Überwachungseinrichtung. Die Überwachungseinrichtung ist mit der Stimulationseinrichtung kommunikativ verbunden. Die Saugvorrichtung und zusätzlich oder alternativ die Spülvorrichtung ist mittels einer Fluidleitung, insbesondere eines semirigiden Schlauchs, über die erste Schnittstelle mit dem Sauginstrument fluidleitend verbunden. Die Stimulationseinrichtung des IOM-Systems ist mittels einer monopolaren oder alternativ einer bipolaren Verbindung, insbesondere eines monopolaren oder alternativ bipolaren Kabels, über die zweite Schnittstelle mit dem Sauginstrument monopolar oder alternativ bipolar elektrisch verbunden. Die Stimulationseinrichtung ist ausgebildet, Gewebe über mindestens eines der beiden Kanülenrohre (monopolare Stimulation), und zusätzlich oder alternativ über beide Kanülenrohre (bipolare Stimulation), elektrisch zu stimulieren. Die Überwachungseinrichtung ist mit der mindestens einen, vorzugsweise mit mindestens zwei Ableitelektroden elektrisch verbunden und ausgebildet, eine von den Ableitelektroden (beispielsweise Nadelelektroden oder Oberflächenelektroden) aufgezeichnete Reizantwort zu überwachen und zusätzlich oder alternativ an einen Anwender akustisch und zusätzlich oder alternativ visuell auszugeben.

Im Rahmen der vorliegenden Erfindung wird der Begriff fluidleitend derart verstanden, dass ein flüssiges oder gasförmiges Fluid dicht entlang einer fluidleitenden Leitung bzw. über eine fluidleitende Schnittstelle gefördert werden kann. Der Begriff dicht ist dabei so zu verstehen, dass keine Leckage auftreten kann, die den Betrieb des Sauginstruments beeinträchtigen würde.

Im Rahmen der vorliegenden Erfindung wird der Begriff elektrisch derart verstanden, dass sowohl ein elektrischer Strom als auch Information über eine elektrische Verbindung bzw. über eine elektrische Schnittstelle übertragen werden kann. Eine elektrische Leitung oder Verbindung bzw. eine elektrische Schnittstelle kann dabei mehrere elektrische Leitungen oder elektrische Verbindungen bzw. elektrische Kontakte (z. B. Stecker oder Buchsen) aufweisen, über die Information nicht nur seriell sondern auch parallel übertragen werden kann.

Im Nachfolgenden werden die Begriffe "proximal" und "distal" wie folgt verwendet. Die Definitionen werden in Bezug zum Anwender gesehen. Distal meint dabei das Ende, das sich weiter weg vom Anwender befindet (das zu einer Spitze des Sauginstruments hin). Proximal meint entsprechend das Ende, das sich näher beim Anwender befindet (hier also das Handstück, das der Anwender in der Hand hat).

In dem vorliegenden Zusammenhang sind chirurgische Zwecke alle Zwecke zu denen das Sauginstrument im Rahmen eines chirurgischen Eingriffs, beispielsweise einer minimalinvasiven Tumorresektion, eingesetzt werden kann.

Das Handstück dient dem Anwender, beispielsweise einem Chirurgen, zur Handhabung des Sauginstruments. Der Anwender kann das Sauginstrument an dem Handstück in die Hand nehmen und führen. Das Handstück kann bevorzugt aus einem Kunststoff gefertigt sein. Zudem kann das Handstück besonders bevorzugt ergonomisch geformt sein, sodass es in der Hand des Anwenders liegt, während dieser das Sauginstrument führt.

Das Handstück ist mechanisch mit der Kanüleneinheit verbunden. Dabei sind Handstück und Kanüleneinheit bevorzugt fest miteinander verbunden, sodass keine Rotation oder Translation gegeneinander möglich ist. Das Handstück und die Kanüleneinheit können über Formschluss und zusätzlich oder alternativ über Kraftschluss und zusätzlich oder alternativ über Stoffschluss miteinander mechanisch verbunden sein. Insbesondere kann dazu das äußere Kanülenrohr der Kanüleneinheit und zusätzlich oder alternativ das innere Kanülenrohr der Kanüleneinheit mit dem Handstück mechanisch verbunden sein, um die mechanische Verbindung zwischen Kanüleneinheit und Handstück bereitzustellen.

Die erste Schnittstelle dient dem Anschluss der externen Saugvorrichtung und zusätzlich oder alternativ der externen Spülvorrichtung an das Sauginstrument. Die erste Schnittstelle kann dazu standardisiert ausgeführt sein. Die erste Schnittstelle kann in Form eines Zapfens oder mehrerer Zapfen ausgeführt sein, an den bzw. an die sich beispielsweise ein semirigider Schlauch mit einer Fluidleitung oder ein semirigider Schlauch, in den zwei Fluidleitungen integriert sind, oder zwei separate semirigide Schläuche anschließen lassen. Über die erste Schnittstelle wird eine fluidleitende Verbindung zwischen dem inneren Kanülenrohr, genauer einem inneren Lumen des inneren Kanülenrohrs, und der Saugvorrichtung und zusätzlich oder alternativ der Spülvorrichtung hergestellt.

Die erste Schnittstelle kann an dem Handstück oder alternativ an der Kanüleneinheit angeordnet sein. Wenn die erste Schnittstelle an der Kanüleneinheit angeordnet ist, dann ist die erste Schnittstelle direkt mit dem inneren Kanülenrohr der Kanüleneinheit bzw. dessen Lumen fluidleitend verbunden. Wenn die erste Schnittstelle an dem Handstück angeordnet ist (beispielsweise an einem proximalen Ende des Handstücks bzw. hinter einer Grifffläche des Handstücks), dann ist die erste Schnittstelle mechanisch (z. B. über eine Steckverbindung) mit dem Handstück verbunden. Alternativ können das Handstück und die erste Schnittstelle auch einstückig bzw. integral ausgeführt sein. Ferner ist die erste Schnittstelle über einen oder mehrere Fluidkanäle in dem Handstück mit dem inneren Kanülenrohr fluidleitend verbunden. Das Handstück kann dazu beispielsweise in seinem Inneren hohl sein und über diesen Hohlraum die fluidleitende Verbindung zwischen der in diesem Beispiel an dem Handstück angeordneten ersten Schnittstelle und dem inneren Kanülenrohr bzw. dessen Lumen herstellen. Der Fluidkanal bzw. die Fluidkanäle (z. B. der Hohlraum im Inneren) des Handstücks sind fluidleitend und dicht mit dem inneren Kanülenrohr verbunden.

Somit kann über die erste Schnittstelle von der angeschlossenen Saugvorrichtung ein Unterdruck bzw. ein Vakuum bereitgestellt werden, mit dem Flüssigkeiten wie beispielsweise Blut und Gewebe wie beispielsweise Tumorgewebe aus dem Situs (Operationsgebiet) abgesaugt werden kann. Zusätzlich oder alternativ kann über die erste Schnittstelle von der angeschlossenen Spülvorrichtung ein Spülmedium wie isotonische Kochsalzlösung mit einem vorbestimmten Druck bereitgestellt werden, sodass der Situs gespült werden kann. Sind die Saugvorrichtung und die Spülvorrichtung gemeinsam an dem Sauginstrument über die erste Schnittstelle fluidleitend angeschlossen, so kann das Gewebe mittels des Spülmediums aus der Spülvorrichtung befeuchtet und gereinigt sowie Verklebungen gelöst und das Spülmedium sowie Blut und Gewebe über den Unterdruck der Saugvorrichtung abgesaugt werden.

Die zweite Schnittstelle dient dem Anschluss der externen Stimulationseinrichtung. Die Schnittstelle kann dazu standardisiert, beispielsweise gemäß DIN 42802 (Touch-Proof-Stecker/Buchse), ausgeführt sein. Die zweite Schnittstelle kann in Form eines oder mehrerer Stecker bzw. einer oder mehrerer Buchsen ausgeführt sein. Dabei kann ein Stecker mehrere Pins und eine Buchse mehrere Aufnahmen für Pins aufweisen. An die zweite Schnittstelle kann ein entsprechendes Kabel angeschlossen werden. Alternativ kann die zweite Schnittstelle selbst mindestens ein, insbesondere zwei oder mehrere solcher Kabel umfassen. Über die zweite Schnittstelle wird eine elektrische Verbindung zwischen dem inneren Kanülenrohr sowie zusätzlich oder alternativ zwischen dem äußeren Kanülenrohr und der Stimulationseinrichtung hergestellt.

Die zweite Schnittstelle kann an dem Handstück oder alternativ an der Kanüleneinheit angeordnet sein. Wenn die zweite Schnittstelle an der Kanüleneinheit angeordnet ist, dann sind der erste Pol der bipolaren elektrischen Verbindung der zweiten Schnittstelle direkt mit dem inneren Kanülenrohr der Kanüleneinheit und der zweite Pol der bipolaren elektrischen Verbindung der zweiten Schnittstelle mit dem äußeren Kanülenrohr der Kanüleneinheit elektrisch verbunden. Wenn die zweite Schnittstelle an dem Handstück angeordnet ist (beispielsweise an einem distalen Ende des Handstücks bzw. vor einer Grifffläche des Handstücks), dann ist der erste Pol der bipolaren elektrischen Verbindung der zweiten Schnittstelle über eine erste elektrische Leitung in oder an dem Handstück mit dem inneren Kanülenrohr und der zweite Pol der bipolaren elektrischen Verbindung der zweiten Schnittstelle über eine zweite elektrische Leitung in oder an dem Handstück mit dem äußeren Kanülenrohr elektrisch verbunden. Die erste und die zweite elektrische Leitung in oder an dem Handstück können beispielsweise als eine kombinierte elektrische Leitung ausgeführt sein.

Somit kann über die zweite Schnittstelle von der angeschlossenen Stimulationseinrichtung an einem der beiden Pole und somit an dem inneren Kanülenrohr (erster Pol) oder dem äußeren Kanülenrohr (zweiter Pol) oder alternativ zwischen beiden Polen und somit zwischen dem inneren und dem äußeren Kanülenrohr ein elektrischer Strom mit einer elektrischen Spannung bereitgestellt werden, mit dem Gewebe, insbesondere Nervengewebe, elektrisch stimuliert werden kann.

Über die sich an dem Patienten in der Nähe des zu überwachenden Nervengewebes angebrachten Ableitelektroden (beispielsweise Nadelelektroden oder Oberflächenelektroden) kann sodann eine Reizantwort des stimulierten (Nerven-)Gewebes aufgenommen und an die elektrisch angeschlossene Überwachungseinrichtung zur weiteren Analyse elektrisch übertragen werden.

Das äußere Kanülenrohr ist elektrisch leitfähig ausgeführt, sodass es als Elektrode zur Stimulation von (Nerven-)Gewebe oder alternativ zum Aufzeichnen bzw. Detektieren der Reizantwort des stimulierten Gewebes dienen kann. Das äußere Kanülenrohr kann zur Umgebung hin elektrisch isoliert (z. B. Beschichtung aus einem elektrisch isolierenden Kunststoff/Polymer, Lack, oder Keramik) sein. Dies ist jedoch bei der bipolaren Stimulation nicht zwingend notwendig, denn dabei fließt der Strom lediglich zwischen den beiden Stimulationskontakten, also von dem inneren Kanülenrohr zu dem äußeren Kanülenrohr oder umgekehrt. Erfolgt eine monopolare Stimulation über das innere Kanülenrohr und eine separate Gegenelektrode (z. B. Nadelelektrode oder Oberflächenelektrode) die an dem Patienten an einer geeigneten Stelle angebracht wurde, kann eine Isolation des äußeren Kanülenrohrs nach außen hin vorliegen. Bei einer monopolaren Stimulation über das äußere Kanülenrohr und die Gegenelektrode muss dagegen eine Isolation des äußeren Kanülenrohrs nach außen hin vorliegen, sodass die Stromabgabe und somit die Stimulation des Gewebes lediglich über die Spitze der Kanüleneinheit und nicht bereits am Schaft bzw. an der Außenfläche des äußeren Kanülenrohrs erfolgt.

Das innere Kanülenrohr ist ebenfalls elektrisch leitfähig ausgeführt, sodass es als Elektrode zur Stimulation von Gewebe oder alternativ zum Aufzeichnen bzw. Detektieren der Reizantwort stimulierten Gewebes dienen kann. Das innere Kanülenrohr ist konzentrisch in dem äußeren Kanülenrohr angeordnet und erstreckt sich von einem proximalen Ende des äußeren Kanülenrohrs bis zu der Spitze (distales Ende) des äußeren Kanülenrohrs. Im Inneren des inneren Kanülenrohrs erstreckt sich ein Lumen, das Fluid, Gewebe oder Gase leiten kann. Das innere Kanülenrohr kann aus dem proximalen Ende des äußeren Kanülenrohrs herausragen und somit in das Innere des Handstückes hineinragen.

Das Innere des Handstücks kann leer (hohl) sein oder in beispielsweise zwei parallel zueinander verlaufende Hohlräume, beispielsweise durch eine sich axial im Handstück erstreckende Trennwand, bis zum Beginn des inneren Kanülenrohrs, aufgeteilt sein. Die Hohlräume stellen separate Fluidleitungen für Fluide bzw. den Unterdruck und das Spülmedium dar. Der eine Hohlraum kann über die erste Schnittstelle und optional über eine entsprechende Fluidleitung im Handstück mit der Saugvorrichtung fluidleitend verbunden sein. Der andere Hohlraum kann über die erste Schnittstelle und optional über eine entsprechende Fluidleitung im Handstück mit der Spülvorrichtung fluidleitend verbunden sein.

Die Isolation befindet sich zwischen dem äußeren und dem inneren Kanülenrohr. Die Isolation kann als einstückiger Schlauch ausgeformt sein, der sich konzentrisch entlang beider Kanülenrohre erstreckt und eine innere Rohroberfläche des äußeren Kanülenrohrs von einer äußeren Rohroberfläche des inneren Kanülenrohrs elektrisch isoliert, um einen elektrischen Kurzschluss zu verhindern. Die Isolation kann auch als ein oder mehrere Abstandshalter (z. B. Ringe) ausgeformt sein, die eine Berührung zwischen der inneren Rohroberfläche des äußeren Kanülenrohrs und der äußeren Rohroberfläche des inneren Kanülenrohrs und somit einen elektrischen Kurzschluss verhindern. Durch die elektrische Isolation der beiden Kanülenrohre können diese als Stimulationselektroden bei der Stimulation von (Nerven-)Gewebe eingesetzt werden.

Das System umfasst neben dem zuvor beschriebenen Sauginstrument auch die Saugvorrichtung und zusätzlich oder alternativ die Spülvorrichtung, die jeweils über die an die erste Schnittstelle angeschlossene Fluidleitung (semirigider Schlauch) mit dem Sauginstrument fluidleitend verbunden sind, die mindestens eine, vorzugsweise zwei Ableitelektroden, sowie das IOM-System.

Das IOM-System umfasst die Stimulationseinrichtung, die über die monopolare oder alternativ bipolare Verbindung (Kabel) an der zweiten Schnittstelle monopolar oder alternativ bipolar angebunden und somit mit dem äußeren Kanülenrohr und dem inneren Kanülenrohr der Kanüleneinheit des Sauginstruments entsprechend monopolar oder alternativ bipolar verbunden ist. Zudem umfasst das IOM-System auch die Überwachungseinrichtung, die kommunikativ mit der Stimulationseinrichtung, beispielsweise über eine Verdrahtung oder ein Bus-System, verbunden ist.

Zur bipolaren Stimulation kann die Stimulationseinrichtung über beide Kanülenrohre Gewebe elektrisch stimulieren. Zur monopolaren Stimulation kann die Stimulationseinrichtung über eines der beiden Kanülenrohre, jedoch bevorzugt über das innere Kanülenrohr aufgrund der flächenmäßig fokussierteren Anregung und Isolation, Gewebe elektrisch stimulieren. Ferner wird eine Gegenelektrode (Nadelelektrode oder Oberflächenelektrode) am Patienten platziert, die die monopolare Stimulation ermöglicht. Über die wenigstens eine, vorzugsweise über zwei Ableitelektroden des Systems kann die Reizantwort des stimulierten Gewebes aufgezeichnet bzw. abgeleitet werden. Die aufgezeichnete Reizantwort des monopolar oder alternativ bipolar stimulierten Gewebes (Nervengewebes) kann als Detektionssignal von der Überwachungseinrichtung aufgenommen, zusätzlich oder alternativ verstärkt und gefiltert werden. Anschließend stellt die Überwachungseinrichtung die, optional verstärkte bzw. gefilterte Reizantwort bzw. das Detektionssignal bereit. Der Anwender, beispielsweise der Chirurg, überwacht die Reizantwort bzw. das Detektionssignal (z. B. abschätzen von Abständen zu Nervengewebe usw.), wobei das Signal beispielsweise visuell oder akustisch beispielsweise auf einem Monitor angezeigt wird. Alternativ oder zusätzlich gibt die Überwachungseinrichtung dem Anwender visuell (z. B. über einen Monitor, auf dem der Verlauf der Reizantwort bzw. des Detektionssignals graphisch dargestellt wird) oder akustisch (z. B. durch einen Signalton dessen Lautstärke mit geringer werdendem Abstand zu Nervengewebe zunimmt) die Reizantwort bzw. das Detektionssignal aus. Die über die Ableitelektroden aufgezeichnete Reizantwort kann bei beiden Stimulationsvarianten als evoziertes Potential und somit als Ableitsignal von der Überwachungseinrichtung aufgenommen, zusätzlich oder alternativ verstärkt und/oder gefiltert werden. Anschließend stellt die Überwachungseinrichtung die, optional gefilterte bzw. verstärkte, Reizantwort bzw. das Ableitsignal beispielsweise visuell auf einem Monitor oder Tablet zur Analyse und Überwachung bereit. Alternativ oder zusätzlich gibt die Überwachungseinrichtung dem Anwender akustisch (z. B. durch einen Signalton) die Gewebestimulation und/oder das EMG-Signal über einen Lautsprecher aus.

Die vorliegende Erfindung stellt somit einen Mappingsauger und ein dieses umfassende System bereit, das Gewebe über die zwei ineinander liegenden Kanülenrohre elektrisch stimuliert. Das Sauginstrument kann sowohl Tumorgewebe als auch Flüssigkeiten absaugen, sodass der Anwender (z. B. Chirurg) eine freie Sicht auf den Situs erreicht. Über die alternative oder zusätzliche Spülfunktion kann zudem der Situs gespült, vor Austrocknung bewahrt und Anhaftungen oder angetrocknete Operationsrückstände gelöst werden. Dies ermöglicht eine sichere Differenzierung zwischen tumorösem und gesundem Gewebe sowie die Nervenlokalisierung im Gewebe und somit eine patientenschonende Resektion. Der bipolare Mappingsauger und das System gemäß der vorliegenden Erfindung können beispielsweise für eine patientenschonende Tumorresektion eingesetzt werden. Die Kombination aus einem chirurgischen Sauger und einer bipolaren Stimulationssonde, die durch das Sauginstrument umgesetzt wird, ermöglicht ein Absaugen von Tumorgewebe und Flüssigkeiten im Situs während einer Tumorresektion, beispielsweise am peripheren und zentralen Nervensystem, und gleichzeitig kontinuierliche, dynamische Identifikation (Mapping) von Nervengewebe, beispielsweise der motorischen Bahnen des zentralen Nervensystems (Pyramidenbahn). Der wesentliche Vorteil der vorliegenden Erfindung ist, dass ein umständliches Wechseln zwischen Stimulationssonde bzw. Stimulationselektrode auf ein Sauginstrument oder Spülinstrument oder die gleichzeitige Anwendung beider Instrumente im Situs nicht mehr nötig ist. Insbesondere eine bipolare Stimulation, also die Anregung des Gewebes über beide Kanülenrohre hat den Vorteil, dass die Anbringung einer zweiten Elektrode als Gegenelektrode (Nadelelektrode oder Oberflächenelektrode) zur Stimulation am Patienten überflüssig ist. Der Strom fließt kontrollierter zwischen den zwei vordefinierten Elektrodenkontakten (inneres und äußeres Kanülenrohr) und breitet sich nicht im umliegenden Gewebe aus, sodass eine sehr fokussierte Stimulation und detaillierte Untersuchung des Gewebes möglich sind.

So kann je nach Bedarf eine bipolare Stimulation oder eine monopolare Stimulation des Gewebes erfolgen und die jeweils optimale Stimulationsart während des chirurgischen Eingriffs angewendet werden.

Vorteilhafte Ausgestaltungen und Weiterbildungen ergeben sich aus den weiteren Unteransprüchen sowie aus der Beschreibung unter Bezugnahme auf die Figuren der Zeichnungen.

Gemäß einer Weiterbildung der vorliegenden Erfindung umfasst das Sauginstrument ferner eine Beleuchtungseinrichtung. Die Beleuchtungseinrichtung umfasst einen Lichtaustritt, einen Lichtleiter und zusätzlich oder alternativ eine Lichtquelle. Der Lichtaustritt ist an dem Handstück oder alternativ an der Kanüleneinheit angeordnet und ausgebildet, Licht von dem Lichtleiter oder alternativ von der Lichtquelle in Richtung eines Situs austreten zu lassen. Der Lichtleiter ist ausgebildet, Licht, das von der Lichtquelle oder alternativ von einer externen Lichtquelle ausgesendet wird, zu dem Lichtaustritt zu leiten.

Der Lichtaustritt ist entweder im Bereich der Spitze der Kanüleneinheit oder an einem distalen Ende des Handstücks angeordnet. Der Lichtaustritt kann eine Linse zum Fokussieren oder Zerstreuen von austretendem Licht umfassen. Der Lichtaustritt kann Licht direkt von einer Lichtquelle, beispielsweise einer an dem distalen Ende des Handstücks angeordneten Lichtquelle, austreten lassen. Alternativ kann der Lichtaustritt Licht, das über den Lichtleiter von der Lichtquelle bzw. der externen Lichtquelle zu dem Lichtaustritt geleitet wurde, austreten lassen.

Der Lichtleiter kann beispielsweise eine Glasfaser oder ein Bündel an Glasfasern sein, entlang welcher Licht der (externen) Lichtquelle geleitet werden kann. Der Lichtleiter kann beispielsweise von dem proximalen Ende der Kanüleneinheit zu deren Spitze (distalem Ende) verlaufen und Licht einer an dem beispielhaft distalen Ende des Handstücks angeordneten Lichtquelle zu der Spitze der Kanüleneinheit leiten. Der Lichtleiter kann sich dabei entlang einer äußeren Rohroberfläche des äußeren Kanülenrohrs erstrecken. Alternativ kann der Lichtleiter sich entlang der inneren Rohroberfläche des äußeren Kanülenrohrs erstrecken, wobei der Lichtleiter in die Isolation eingebettet sein kann und optional sich auch entlang der äußeren Rohroberfläche des inneren Kanülenrohrs erstrecken kann. Zudem alternativ kann der Lichtleiter sich entlang der äußeren Rohroberfläche des inneren Kanülenrohrs erstrecken, wobei der Lichtleiter in die Isolation eingebettet sein kann. Weiter alternativ kann der Lichtleiter sich entlang der inneren Rohroberfläche des inneren Kanülenrohrs erstrecken. Alternativ kann sich der Lichtleiter von einer externen Lichtquelle über das Handstück und/oder die Kanüleneinheit zu dem Lichtaustritt hin erstrecken und so extern erzeugtes Licht zu dem Lichtaustritt leiten.

Der Lichtleiter ist mit der Lichtquelle bzw. der externen Lichtquelle optisch verbunden. Entweder ist die Lichtquelle von dem Sauginstrument umfasst und beispielsweise in das Handstück integriert, oder sie ist eine externe Lichtquelle. Die externe Lichtquelle kann eine eigenständige externe Einrichtung oder von einer externen Einrichtung beispielsweise von der Stimulationseinrichtung bzw. vom IOM-System umfasst sein. Die von dem Sauginstrument umfasste Lichtquelle kann beispielsweise über die zweite Schnittstelle mit elektrischem Strom versorgt werden. Die Lichtquelle bzw. die externe Lichtquelle kann bevorzugt eine LED sein. Diese kann mit einer Stromquelle elektrisch verbunden sein, wobei die Stromquelle beispielsweise von der Stimulationseinrichtung bzw. vom IOM-System umfasst ist und die LED mit Strom versorgt. Die LED kann direkt am oder im Handstück integriert sein, vorzugsweise an dessen distalem Ende. Alternativ kann die LED direkt an der Kanüleneinheit angeordnet sein.

Mit dem direkt aus der Lichtquelle oder aus dem Lichtleiter an dem Lichtaustritt austretenden Licht kann der Situs besser ausgeleuchtet werden, sodass der Anwender bzw. Chirurg eine verbesserte Sicht auf die im Situs befindlichen Strukturen und Gewebe hat und diese besser differenzieren kann.

Gemäß einer Weiterbildung der vorliegenden Erfindung umfasst das Sauginstrument ferner ein Trackingelement. Das Trackingelement ist eingerichtet, von einer externen Navigationsvorrichtung erkannt zu werden. Das Trackingelement ist fest oder lösbar mechanisch mit dem Handstück oder der Kanüleneinheit verbunden.

Das Trackingelement kann ein aktives (z. B. Leuchtdioden, elektromagnetisches Element) oder passives Trackingelement (z. B. optische Marker) sein. Das Trackingelement umfasst, mindestens einen, bevorzugt drei (aktive oder passive) Marker, die in einer vordefinierten Geometrie zueinander angeordnet sind (z. B. drei Trackingkugeln in einem vordefinierten ungleichmäßigen Dreieck an dem Trackingelement angeordnet). Die Marker können von der Navigationsvorrichtung (z. B. Stereokamera, zwei elektromagnetische Sensoren usw.) im Raum erkannt werden. Aus der Lage und Pose (Position und Orientierung) der Marker kann das Navigationssystem die relative Lage und Pose des Sauginstruments in Bezug zum Patienten und zu registrierten Patientenbilddaten anzeigen.

Das Trackingelement kann mit dem Handstück oder direkt mit der Kanüleneinheit, beispielsweise durch Anschrauben oder durch einen Schnappmechanismus, lösbar mechanisch verbunden oder alternativ fest bzw. starr mechanisch mit dem beispielsweise proximalen Ende des Handstückes oder an der Kanüleneinheit verbunden sein.

Durch das Trackingelement kann dem Anwender bzw. Chirurgen die Lage und Pose des Sauginstruments im Bezug zum Patienten und in den registrierten Bilddaten des Patienten angezeigt werden, wodurch, auch wenn der Situs nicht von außen eingesehen werden kann, die Lage und Pose der Spitze des Sauginstruments im Situs, also innerhalb des Patienten, für den Anwender bzw. Chirurgen erkennbar sind.

Gemäß einer Weiterbildung der vorliegenden Erfindung sind das äußere Kanülenrohr und optional das innere Kanülenrohr und zusätzlich oder alternativ die Isolation biokompatibel oder bioinert ausgeführt. Insbesondere sind das äußere Kanülenrohr und zusätzlich oder alternativ das innere Kanülenrohr aus Edelstahl und zusätzlich oder alternativ die Isolation aus Kunststoff, insbesondere Polyamid, gefertigt.

Die biokompatible Ausführung stellt sicher, dass die wenigen von dem äußeren Kanülenrohr bzw. dem inneren Kanülenrohr bzw. der Isolation abgegebenen Stoffe (z. B. Ionen) keine negative Reaktion (z. B. Allergie, Änderung des pH-Werts, Vergiftung usw.) im Situs bzw. dem Patienten auslösen. Die bioinerte Ausführung sorgt dafür, dass keinerlei Stoffe von dem äußeren Kanülenrohr bzw. dem inneren Kanülenrohr bzw. der Isolation in den Situs abgegeben werden.

Gemäß einer Weiterbildung der vorliegenden Erfindung weist die Kanüleneinheit einen äußeren Durchmesser von 1 mm [Millimeter] bis 15 mm auf.

Je nach Art des chirurgischen Eingriffs und Lage des Situs am Patienten können verschiedene Durchmesser der Kanüleneinheit bzw. des äußeren Kanülenrohrs angezeigt sein.

Das äußere Kanülenrohr und zusätzlich oder alternativ das innere Kanülenrohr können eine Wandstärke von 1% [Prozent] bis 10% des äußeren Durchmessers der Kanüleneinheit aufweisen. Dabei können das äußere Kanülenrohr und das innere Kanülenrohr die gleiche Wandstärke oder jeweils eine unterschiedliche Wandstärke aufweisen.

Durch die Wandstärke lässt sich die Fläche des äußeren und inneren Kanülenrohrs, die mit dem Gewebe in Kontakt kommt, an die für die Stimulation von Gewebe und Aufzeichnung bzw. Detektion der Reizantworten optimalen elektrischen Eigenschaften anpassen.

Gemäß einer Weiterbildung der vorliegenden Erfindung weist die Kanüleneinheit eine Länge von 10 cm [Zentimeter] bis 40 cm auf.

Je nach Art des chirurgischen Eingriffs und Lage des Situs am Patienten können verschiedene Längen der Kanüleneinheit, d.h. des äußeren und inneren Kanülenrohrs, angezeigt sein.

Gemäß einer Weiterbildung der vorliegenden Erfindung ist die Kanüleneinheit gerade ausgeführt oder weist in einem proximalen Bereich eine Abwinklung mit einem Winkel von 10° [Grad] bis 60°, bevorzugt von 30° auf.

Je nach Art des chirurgischen Eingriffs und Lage des Situs am Patienten kann die Kanüleneinheit bzw. die beiden Kanülenrohre nicht nur gerade sondern am proximalen Ende nahe am Handstück unter einem Winkel, bevorzugt von 30°, gebogen sein, sodass die Sicht des Anwenders auf die Strukturen und das Gewebe im Situs nicht durch seine eigene Hand beeinträchtigt wird.

Gemäß einer Weiterbildung der vorliegenden Erfindung umfasst das Handstück eine Saugkontrollöffnung, die mit dem inneren Kanülenrohr fluidleitend verbunden ist.

Die Saugkontrollöffnung ist mit dem Lumen des inneren Kanülenrohrs über entsprechende Fluidleitungen im Handstück fluidleitend verbunden. Beispielsweise kann die Saugkontrollöffnung mit dem Hohlraum in dem Handstück, der die erste Schnittstelle mit dem inneren Kanülenrohr bzw. dessen Lumen verbindet, fluidleitend verbunden sein. Zudem kann die Saugkontrollöffnung mit einem Finger, bevorzugt dem Daumen, der Anwenders dicht verschlossen werden. Alternativ ist es auch möglich, die Saugkontrollöffnung mit einem (fingerbetätigbaren) Schieber oder Klappe zu verschließen.

Bevorzugt befindet sich die Saugkontrollöffnung an einer Oberseite des Handstücks.

Ist die Saugkontrollöffnung frei und nicht abgedeckt, reduziert sich der Unterdruck bzw. das Vakuum an der Spitze (distalem Ende) der Kanüleneinheit, wodurch die Saugkraft verringert wird. Je weiter die Saugkontrollöffnung (mit dem Finger) verschlossen wird, desto größer wird die Saugkraft an der Spitze der Kanüleneinheit wieder. Die Saugkontrollöffnung dient somit der Kontrolle der Saugintensität. Während des Saugvorganges kann der Anwender die Saugkontrollöffnung (mit dem Daumen) verschließen und die Saugwirkung an der Spitze der Kanüleneinheit entsprechend verstärken. Wird während der Operation beispielsweise unerwünschtes Gewebe (z. B. Nervengewebe) von der Spitze der Kanüleneinheit angesaugt, kann durch Öffnen der Saugkontrollöffnung (Heben des Daumens) der Sog an der Spitze reduziert werden und das Gewebe an der Spitze fällt ab.

Gemäß einer Weiterbildung ist die Kanüleneinheit an dem proximalen Ende der Kanüleneinheit fest oder lösbar mechanisch mit dem Handstück verbunden.

Die Kanüleneinheit kann beispielsweise in das Handstück eingeschraubt oder über einen Schnappverschluss oder einen Bajonett-verschluss oder einen Luer-Lock in bzw. an dem Handstück gesichert und somit lösbar mechanisch mit diesem verbunden werden. Alternativ kann die Kanüleneinheit fest mechanisch, beispielsweise über eine Verklebung oder durch Eingießen in das Handstück, mit dem Handstück verbunden sein. Sollten in dem Handstück Fluidleitungen und zusätzlich oder alternativ elektrische Leitungen für die Verbindung des ersten und zweiten Kanülenrohrs mit der ersten bzw. zweiten Schnittstelle vorhanden sein, so sind entsprechende Dichtungen bzw. Kontakte an dem Handstück vorgesehen, um die fluidleitenden bzw. elektrischen Verbindungen von dem äußeren und inneren Kanülenrohr zu der ersten bzw. zweiten Schnittstelle sicherzustellen.

Dies hat den Vorteil, insbesondere wenn die erste und zweite Schnittstelle direkt an der Kanüleneinheit angeordnet sind, dass das Handstück besonders einfach (ohne elektrische Leitungen oder Fluidkanäle) ausgestaltet sein kann. Zudem können vorhandene Standardhandstücke mit der Kanüleneinheit gemäß der vorliegenden Erfindung nachgerüstet werden. Außerdem lassen sich das Handstück und die Kanüleneinheit nach dem chirurgischen Eingriff voneinander trennen und dann getrennt reinigen sowie beispielsweise einerseits die Kanüleneinheit sterilisieren und das Handstück lediglich desinfizieren.

Das Sauginstrument mit dessen Einzelteilen kann zur einmaligen oder mehrmaligen Verwendung ausgelegt sein. Die zur Fertigung verwendeten Materialien sind folglich auch für eine etwaige Wiederaufbereitung mit beispielsweise Ethylenoxid vorgesehen.

Gemäß einer Weiterbildung der vorliegenden Erfindung umfasst das Sauginstrument ferner ein erstes steuerbares Ventil und ein zweites steuerbares Ventil. Das innere Kanülenrohr ist über das erste steuerbare Ventil und einen ersten Fluidanschluss der ersten Schnittstelle mit der externen Saugvorrichtung fluidleitend verbunden. Zusätzlich oder alternativ ist das innere Kanülenrohr über das zweite steuerbare Ventil und einen zweiten Fluidanschluss der ersten Schnittstelle mit der externen Spülvorrichtung fluidleitend verbunden.

Die steuerbaren Ventile sind als steuerbare Sperrventile oder Stromventile ausgestaltet. Beide steuerbaren Ventile können entweder in Ruhelage sperren/schließen und bei Betätigung freigeben/öffnen oder umgekehrt in Ruhelage freigeben/öffnen und bei Betätigung sperren/schließen. Die steuerbaren Ventile können mechanisch (z. B. über einen Knopf, einen Schalter, einen Hebel, einen Dreh-Hahn usw.) und zusätzlich oder alternativ elektrisch (über eine Schaltung mit entsprechender Verdrahtung) und zusätzlich oder alternativ magnetisch (integrierter oder externer schaltbarer Elektromagnet) betätigbar, also von einer geöffneten Stellung in eine geschlossene Stellung und umgekehrt verschiebbar, sein.

Durch das erste und zweite steuerbare Ventil kann die Saugvorrichtung bzw. die Spülvorrichtung gezielt zugeschaltet oder abgeschaltet und so je nach Bedarf der Situs abgesaugt oder gespült werden.

Gemäß einer Weiterbildung der vorliegenden Erfindung umfasst das Handstück ferner ein erstes Bedienelement. Das erste Bedienelement ist ausgebildet, zwischen einer Saugfunktion der externen Saugvorrichtung und einer Spülfunktion der externen Spülvorrichtung umzuschalten.

Das erste Bedienelement ist dabei ausgebildet, um zwischen der Saugfunktion und der Spülfunktion gezielt umzuschalten, sodass je nach Bedarf Gewebe oder Flüssigkeiten abgesaugt oder der Situs gespült werden kann. Das Sauginstrument wird durch Betätigen des ersten Bedienelements entweder zum Absaugen von Fluid, Gewebe oder Gase oder zum Spülen des Situs nutzbar gemacht.

Das Sauginstrument kann dazu beispielsweise das erste steuerbare Ventil und das zweite steuerbare Ventil umfassen. Dabei ist das erste Bedienelement ausgebildet, das erste steuerbare Ventil zu öffnen und gleichzeitig das zweite steuerbare Ventil zu schließen sowie das erste steuerbare Ventil zu schließen und gleichzeitig das zweite steuerbare Ventil zu öffnen. Optional kann das erste Bedienelement zusätzlich ausgebildet sein, beide steuerbaren Ventile zu schließen.

Alternativ können die Ventile oder ein Ventil beispielsweise an der fluidleitenden Verbindung der ersten Schnittstelle zur Saugvorrichtung und/oder Spülvorrichtung integriert sein. Weiter alternativ können über das erste Bedienelement auch die externe Saugvorrichtung und die externe Spülvorrichtung gesteuert, nämlich eingeschaltet und ausgeschaltet werden. Dazu ist das erste Bedienelement kommunikativ, beispielsweise über die erste oder zweite Schnittstelle, mit der externen Saugvorrichtung und/oder der externen Spülvorrichtung verbunden. In diesen beispielhaften Ausführungen kann auf steuerbare Ventile im oder am Handstück verzichtet werden.

Das erste Bedienelement kann beispielsweise als Schalter auf dem Handstück oder alternativ an der Kanüleneinheit ausgestaltet sein, der beispielsweise das erste steuerbare Ventil und das zweite steuerbare Ventil mechanisch ansteuert oder die externe Saugvorrichtung und die externe Spülvorrichtung kommunikativ steuert.

Der Anwender kann das erste Bedienelement (Schalter) beispielsweise mit einem Finger betätigen. Bevorzugt befindet sich das erste Bedienelement auf einer seitlichen Fläche des Handstücks und ist mit dem Daumen oder dem Zeigefinger betätigbar.

Das erste Bedienelement kann in eine erste Stellung (z. B. Schalter ganz nach oben/rechts geklappt) und in eine zweite Stellung (z. B. Schalter ganz nach unten/links geklappt) bewegt werden. In der ersten Stellung kann das erste Bedienelement die Saugfunktion einschalten und die Spülfunktion ausschalten und somit das Absaugen von Fluid ermöglichen, wobei das gleichzeitige Spülen des Situs verhindert wird. Dies kann beispielsweise durch Einschalten der externen Saugvorrichtung und Ausschalten der externen Spülvorrichtung durch das erste Bedienelement erfolgen. Alternativ kann das erste Bedienelement das erste steuerbare Ventil öffnen und das zweite steuerbare Ventil gleichzeitig schließen. In der zweiten Stellung kann das erste Bedienelement die Saugfunktion ausschalten und die Spülfunktion einschalten und somit das Spülen mittels Spülflüssigkeit ermöglichen, wobei das gleichzeitige Absaugen des Situs verhindert wird. Dies kann beispielsweise durch Ausschalten der externen Saugvorrichtung und Einschalten der externen Spülvorrichtung durch das erste Bedienelement erfolgen. Alternativ kann das erste Bedienelement das erste steuerbare Ventil schließen und das zweite steuerbare Ventil gleichzeitig öffnen. Folglich ist durch Bedienen des Schalters immer nur eine Funktion ausführbar, entweder Absaugen oder Spülen.

Optional kann das erste Bedienelement zusätzlich in eine dritte Stellung (z. B. Schalter in eine mittlere oder neutrale Stellung geklappt) bewegt werden. In der dritten Stellung kann das erste Bedienelement die externe Saugvorrichtung und die externe Spülvorrichtung ausschalten oder alternativ das erste steuerbare Ventil schließen und gleichzeitig das zweite steuerbare Ventil schließen. Weder Absaugen noch Spülen ist möglich, sodass nur eine reine monopolare oder bipolare Stimulation über das Sauginstrument erfolgen kann.

Mit dem ersten Bedienelement sind die Saugvorrichtung und die Spülvorrichtung zu- und abschaltbar, sodass der Situs besonders einfach und schnell entweder abgesaugt oder gespült werden kann (oder keines von beiden).

Gemäß einer Weiterbildung der vorliegenden Erfindung umfasst die zweite Schnittstelle zwei Anschlusspins zur Herstellung der monopolaren oder bipolaren Verbindung. Alternativ umfasst die zweite Schnittstelle ein bipolares Kabel zur Herstellung der monopolaren oder bipolaren elektrischen Verbindung.

Die zwei Anschlusspins sind nicht isolierte, elektrisch leitende Vorsprünge, die eine im Wesentlichen zylindrische Form aufweisen können. Bevorzugt sind die zwei Anschlusspins als Touch-Proof-Stecker, die besonders bevorzugt gemäß DIN 42802 ausgestaltet sind. Einer der zwei Anschlusspins ist für den ersten Pol der bipolaren elektrischen Verbindung und der andere der zwei Anschlusspins ist für den zweiten Pol der bipolaren elektrischen Verbindung vorgesehen. Alternativ sind eine monopolare Verbindung und somit eine monopolare Stimulation durch Anschluss eines monopolaren Kabels an nur einen der beiden Anschlusspins, vorzugsweise an den ersten Pol, der mit dem inneren Kanülenrohr der Kanüleneinheit verbunden ist, möglich.

Die zwei Anschlusspins können an dem Handstück, bevorzugt an einer Unterseite des Handstücks und besonders bevorzugt distal an der Unterseite des Handstücks angeordnet und über entsprechende elektrische Verbindungen, beispielsweise über Lötstellen oder Schweißstellen, mit dem inneren Kanülenrohr und dem äußeren Kanülenrohr elektrisch verbunden sein. Alternativ können die zwei Anschlusspins direkt an der Kanüle, bevorzugt an einer Unterseite der Kanüleneinheit und besonders bevorzugt proximal an der Unterseite der Kanüleneinheit angeordnet und direkt mit dem inneren Kanülenrohr und dem äußeren Kanülenrohr elektrisch verbunden sein. An die beiden Anschlusspins kann jeweils ein Kabel für eine bipolare Stimulation oder alternativ bei Bedarf einer monopolaren Stimulation nur ein Kabel an ein Anschlusspin zur elektrischen Verbindung mit der Stimulationseinrichtung angeschlossen werden.

Der Vorteil hierbei ist, dass das Sauginstrument bei Bedarf (z. B. Reinigung und Sterilisation) vom jeweiligen Anschlusskabel getrennt werden kann.

Statt den zwei Anschlusspins kann ein bipolares Kabel vorgesehen sein. Das bipolare Kabel kann an seinem Ende, entweder zwei Stecker bzw. zwei Pins in einem gemeinsamen Stecker oder zwei Buchsen bzw. zwei Aufnahmen für je einen Pin in einer gemeinsamen Buchse aufweisen, die bevorzugt Touch-Proof und besonders bevorzugt gemäß DIN 42802 ausgeführt sind. Das bipolare Kabel ist entweder direkt an dem Handstück, bevorzugt an einer Unterseite des Handstücks, besonders bevorzugt distal an der Unterseite des Handstücks angeschlossen und beispielsweise über entsprechende elektrische Verbindungen, beispielsweise über Lötstellen oder Schweißstellen, mit dem äußeren Kanülenrohr und dem inneren Kanülenrohr elektrisch verbunden. Oder das bipolare Kabel ist alternativ direkt an der Kanüleneinheit, bevorzugt an einer Unterseite der Kanüleneinheit und besonders bevorzugt proximal an der Unterseite der Kanüleneinheit angeschlossen und direkt mit dem äußeren Kanülenrohr und dem inneren Kanülenrohr elektrisch verbunden. An das bipolare Kabel kann die Stimulationseinrichtung angeschlossen werden. Wenn das bipolare Kabel von der zweiten Schnittstelle umfasst ist, dann kann nur eine(r) von zwei Buchsen oder Steckern des bipolaren Kabels an der Stimulationseinrichtung eingesteckt werden, um eine monopolare Stimulation zu ermöglichen.

Der Vorteil ist, dass das Kabel vor dem Einsatz nicht erst noch am Sauginstrument angeschlossen werden muss, was Zeit spart.

Die zwei Anschlusspins oder das bipolare Kabel ermöglichen es, das Sauginstrument besonders einfach und schnell mit der Stimulationseinrichtung monopolar oder bipolar elektrisch zu verbinden.

Gemäß einer Weiterbildung der vorliegenden Erfindung umfasst das Sauginstrument ferner ein zweites Bedienelement. Die zweite Schnittstelle ist ferner ausgebildet, eine kommunikative Verbindung des zweiten Bedienelements mit der externen Stimulationseinrichtung herzustellen. Das zweite Bedienelement ist ausgebildet, zwischen einem monopolaren Betrieb und einem bipolaren Betrieb umzuschalten.

Das zweite Bedienelement kann ein Schalter oder der geleichen sein. Das zweite Bedienelement ist in zwei oder alternativ drei Stellungen bewegbar (z. B. Schalter ganz nach oben/rechts geklappt Schalter ganz nach unten/links geklappt und Schalter in eine mittlere oder neutrale Stellung geklappt). In der ersten Stellung kann das zweite Bedienelement der Stimulationseinrichtung über die zweite Schnittstelle kommunikativ mitteilen (z. B. steigende Flanke usw.), dass der bipolare Betrieb durchzuführen ist, in dem das Gewebe über einen Stromfluss zwischen beiden Kanülenrohren stimuliert wird. In der zweiten Stellung kann das zweite Bedienelement der Stimulationseinrichtung über die zweite Schnittstelle kommunikativ mitteilen (z. B. fallende Flanke usw.), dass der monopolare Betrieb durchzuführen ist, in dem eines der beiden Kanülenrohre Gewebe stimuliert. Bei einer monopolaren Stimulation wird eine externe Gegenelektrode am Patienten nahe des zu stimulierenden (Nerven-)Gewebes befestigt und der elektrische Strom bzw. die elektrische Spannung zwischen dem entsprechenden Kanülenrohr und der externen Gegenelektrode aufgebracht. Die Reizantwort des bipolar oder monopolar stimulierten Gewebes wird über zusätzlich am Patienten angebrachte Ableitelektroden aufgezeichnet bzw. abgeleitet. In der dritten Stellung des zweiten Bedienelements kann die Stimulationseinrichtung nicht (weder monopolar noch bipolar) stimulieren, sodass nur eine reine Absaugung von Gewebe und Fluid oder eine Spülung des Situs möglich ist.

Mit dem zweiten Bedienelement kann zwischen dem bipolaren und dem monopolaren Betrieb je nach Bedarf umgeschaltet werden, sodass schnell und einfach die optimale Betriebsart von dem Anwender ausgewählt werden kann. Zusätzlich kann schnell die Stimulation von Gewebe ganz abgeschaltet werden.

Gemäß einer Weiterbildung der vorliegenden Erfindung umfasst das System entweder ferner ein Impedanzmessgerät, das mit der Überwachungseinheit kommunikativ verbunden und ausgebildet ist, eine Impedanzmessung durchzuführen, oder alternativ ist das IOM-System ausgebildet, die Impedanzmessung durchzuführen. Zur Impedanzmessung werden komplexe Widerstände bei verschiedenen Frequenzen von an der Spitze der Kanüleneinheit umliegendem Gewebe gemessen. Das System, insbesondere die Überwachungseinheit, ist ausgebildet, basierend auf den gemessenen Impedanzen die Art des Gewebes zu bestimmen und an den Nutzer auszugeben.

Es ist möglich, über die Kanüleneinheit des Sauginstruments eine Impedanzspektroskopie durchzuführen. Während eines chirurgischen Eingriffs kann die elektrische Impedanz bei verschiedenen Frequenzen von dem an der Spitze der Kanüleneinheit umliegenden Gewebe gemessen und so die Art des Gewebes bestimmt werden. Hierbei geht es um die Differenzierung von beispielsweise Knochen, Nervengewebe und/oder Haut und vorzugsweise um die Differenzierung von Tumorgewebe und gesundem Gewebe, beispielsweise im Gehirn. Die Impedanz des Gewebes wird dabei beispielsweise innerhalb eines ringförmigen Volumens an der unisolierten Spitze der Kanüleneinheit gemessen, wobei die Verteilung des Stroms von den Gewebeeigenschaften abhängt. Dabei werden Wechselströme wird ein elektrischer Strom mit verschiedenen, vordefinierten Frequenzen in das Gewebe eingeleitet. Abhängig von der jeweiligen Frequenz weisen unterschiedliche Arten von Gewebe jeweils eine andere Impedanz auf. Die Impedanzen bei den verschiedenen, vordefinierten Frequenzen sind charakteristisch für verschiedene Arten von Gewebe.

Bei der Impedanzspektroskopie wird ein elektrisches Wechselsignal, beispielsweise ein vordefinierter Wechselstrom bzw. eine vordefinierte Wechselspannung, beispielsweise aber nicht ausschließlich in Form eines Rechtecksignals oder eines Sinussignals, über eines der Kanülenrohre an der Spitze der Kanüleneinheit auf das Gewebe gegeben. Das Impedanzmessgerät oder das IOM-System misst anschließend entsprechend die Spannung bzw. den Strom an dem anderen Kanülenrohr und berechnet über eine entsprechende Signalverarbeitungskette und/oder Software die Impedanz des Gewebes.

Durch die Möglichkeit der Impedanzspektroskopie über das Sauginstrument ist auch hier kein Wechseln der Instrumente notwendig, da die Signalabgabe ebenfalls über das System, das die monopolare und/oder bipolare Stimulation abgibt, erfolgt. Bei einem bipolaren Aufbau konzentriert sich der Strom- bzw. Spannungspfad vorteilhaft auf den Bereich zwischen den beiden Stimulationspolen, wodurch die Impedanzmessung größtenteils lediglich durch das lokale Gewebe beeinflusst wird, wohingegen bei einem monopolaren Aufbau der Strom- bzw. Spannungspfad durch das für die Untersuchung nicht relevante Gewebe zu einer am Patienten angebrachten Gegenelektrode (beispielsweise Nadelelektrode oder Oberflächenelektrode) geleitet wird, sodass die Messung der Impedanz beeinflusst werden könnte.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird dabei der Fachmann auch Einzelaspekte als Verbesserungen oder Ergänzungen zu der jeweiligen Grundform der vorliegenden Erfindung hinzufügen.

### INHALTSANGABE DER ZEICHNUNGEN

Die vorliegende Erfindung wird nachfolgend anhand der in den schematischen Figuren der Zeichnung angegebenen Ausführungsbeispiele näher erläutert. Es zeigen dabei:
- Fig. 1: eine schematische Ansicht des Sauginstruments gemäß dem ersten Aspekt der vorliegenden Erfindung,
- Fig. 2: eine vergrößerte schematische Ansicht der Kanüleneinheit des Sauginstruments aus Fig. 1,
- Fig. 3: eine schematische Ansicht einer weiteren Ausführungsform des Sauginstruments gemäß dem ersten Aspekt der vorliegenden Erfindung,
- Fig. 4: eine schematische Ansicht einer weiteren Ausführungsform des Sauginstruments gemäß dem ersten Aspekt der vorliegenden Erfindung,
- Fig. 5: eine schematische Ansicht einer weiteren Ausführungsform des Sauginstruments gemäß dem ersten Aspekt der vorliegenden Erfindung und
- Fig. 6: eine schematische Ansicht des Systems gemäß dem zweiten Aspekt der vorliegenden Erfindung.

Die beiliegenden Figuren der Zeichnung sollen ein weiteres Verständnis der Ausführungsformen der Erfindung vermitteln.

Sie veranschaulichen beispielhafte Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktions-gleiche und gleich wirkende Elemente, Merkmale und Komponenten - sofern nichts anderes ausgeführt ist - jeweils mit denselben Bezugszeichen versehen.

### BESCHREIBUNG VON AUSFÜHRUNGSBEISPIELEN

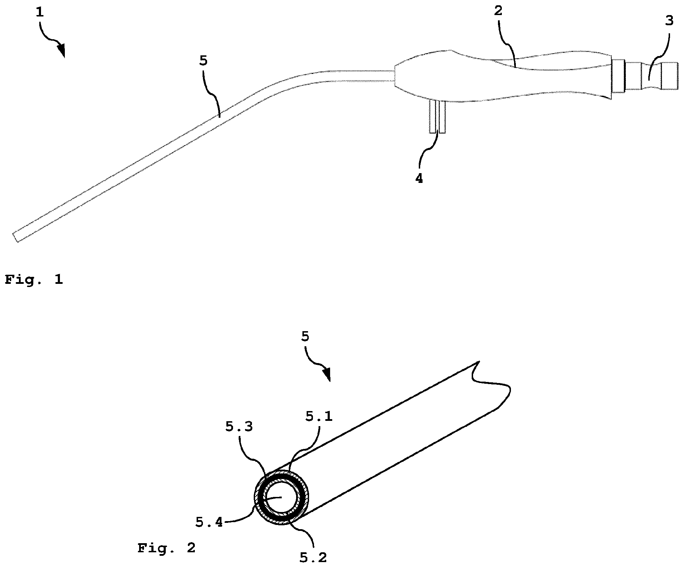

In Fig. 1 ist das Sauginstrument 1, hier ein bipolarer Mappingsauger, gemäß dem ersten Aspekt der vorliegenden Erfindung schematisch dargestellt. Das Sauginstrument 1 umfasst ein Handstück 2, eine erste Schnittstelle 3, eine zweite Schnittstelle 4 und eine Kanüleneinheit 5.

Das Handstück 2 ist ergonomisch geformt und aus Kunststoff (z. B. Poyetheretherketon, PEEK, PC/ABS) hergestellt. Ein Chirurg kann das Handstück 2 in die Hand nehmen und darüber das Sauginstrument 1 während eines chirurgischen Eingriffs führen.

Die erste Schnittstelle 3 ist als fluidleitende Kupplung für einen semirigiden Schlauch (nicht dargestellt) an einem proximalen Ende des Handstücks 2 ausgeformt. An die erste Schnittstelle 3 kann über den semirigiden Schlauch sowohl eine Saugvorrichtung (nicht dargestellt) als auch eine Spülvorrichtung (nicht dargestellt) fluidleitend angeschlossen werden.

Die zweite Schnittstelle 4 ist in Form von zwei Anschlusspins distal an einer Unterseite des Handstücks 2 ausgeführt, an welche ein bipolares Kabel (nicht dargestellt) angeschlossen werden kann, und umfasst eine bipolare Verbindung. An die zweite Schnittstelle 4 kann über das bipolare Kabel eine Stimulationseinrichtung (nicht dargestellt) elektrisch angeschlossen und somit bipolar verbunden werden. Alternativ kann ein monopolares Kabel an einen der Anschlusspins angeschlossen werden.

Die Kanüleneinheit 5 ist mit ihrem proximalen Ende mit dem Handstück 2 fest mechanisch verbunden. Die Kanüleneinheit 5 erstreckt sich zu einer Spitze bzw. einem distalen Ende der Kanüleneinheit 5 hin. Dabei weist die Kanüleneinheit 5 eine Abwinklung in einem proximalen Bereich der Kanüleneinheit 5 von 30° nach unten auf.

Der genaue Aufbau der Kanüleneinheit 5 ist in der Fig. 2 schematisch dargestellt. Die Kanüleneinheit 5 umfasst ein äußeres Kanülenrohr 5.1, ein inneres Kanülenrohr 5.2 und eine Isolierung 5.3.

Das äußere Kanülenrohr 5.1 ist aus Edelstahl geformt und mit dem Handstück 2 lösbar fest mechanisch verbunden (z. B. Verschraubung, Schnappverbindung usw.). Das äußere Kanülenrohr erstreckt sich von dem Handstück 2 bis zu der Spitze der Kanüleneinheit 5. Ferner ist das äußere Kanülenrohr 5.1 mechanisch mit dem Handstück 2 und über einen elektrischen Kontakt, beispielsweise einer Lötstelle oder Schweißstelle, in dem Handstück 2 mit einem Pol der bipolaren Verbindung der zweiten Schnittstelle 4 und somit mit der Stimulationseinrichtung elektrisch verbunden. Somit kann das äußere Kanülenrohr 5.1, insbesondere an der Spitze der Kanüleneinheit 5, als Elektrode für eine monopolare oder bipolare Stimulation von Gewebe durch die Stimulationseinrichtung verwendet werden.

Das innere Kanülenrohr 5.2 ist aus Edelstahl geformt und verläuft konzentrisch zu dem äußeren Kanülenrohr 5.1. Dabei erstreckt sich das innere Kanülenrohr 5.2 von der Spitze der Kanüleneinheit 5 bis über ein proximales Ende des äußeren Kanülenrohrs 5.1 hinaus in das Handstück 2 hinein. Das innere Kanülenrohr 5.2 ist mechanisch mit dem Handstück 2 und über einen elektrischen Kontakt, beispielsweise einer Lötstelle oder Schweißstelle, in dem Handstück 2 mit dem entsprechend anderen Pol der bipolaren Verbindung der zweiten Schnittstelle 4 und somit mit der Stimulationseinrichtung elektrisch verbunden. Somit kann das innere Kanülenrohr 5.2 als Elektrode für eine monopolare oder bipolare Stimulation von Gewebe durch die Stimulationseinrichtung verwendet werden.

Die Isolierung 5.3 ist als Schlauch aus Polyamid ausgeformt und isoliert das äußere Kanülenrohr 5.1 über dessen gesamte Länge von dem darin konzentrisch verlaufenden inneren Kanülenrohr 5.2. Dadurch wird ein elektrischer Kurzschluss zwischen den beiden Kanülenrohren 5.1, 5.2 vermieden und eine bipolare Stimulation von Gewebe durch die Steuerungseinrichtung ermöglicht.

Das innere Kanülenrohr 5.2 weist ein Lumen 5.4 auf, das sich von der Spitze der Kanüleneinheit 5 bis zu einem distalen Ende des inneren Kanülenrohrs 5.2 erstreckt. Das Lumen 5.4 des inneren Kanülenrohrs 5.2 ist mechanisch in dem Handstück 2 und dadurch mit der ersten Schnittstelle 3 und somit mit der Saugvorrichtung und der Spülvorrichtung fluidleitend verbunden. Das Innere des Handstückes 2 kann zweigeteilt ausgeformt sein, sodass ein erster Hohlraum (nicht dargestellt) durch eine Trennwand (nicht dargestellt) von einem zweiten Hohlraum (nicht dargestellt) bis zum Beginn des inneren Kanülenrohrs 5.2 getrennt wird. Die Saugvorrichtung kann beispielsweise mit dem ersten Hohlraum fluidleitend verbunden sein, während die Spülvorrichtung mit dem zweiten Hohlraum fluidleitend verbunden sein kann. Durch die Saugvorrichtung kann somit ein Unterdruck bzw. Vakuum an das Lumen 5.4 des inneren Kanülenrohrs 5.2 angelegt werden, sodass an der Spitze der Kanüleneinheit 5 eine Saugwirkung erzeugt wird, mit der Flüssigkeiten wie Blut und Gewebe aus dem Situs abgesaugt werden können. Analog kann durch die Spülvorrichtung somit ein Spülmedium wie isotonische Kochsalzlösung zu der Spitze der Kanüleneinheit 5 gefördert und der Situs damit gespült bzw. befeuchtet werden. Das Spülmedium der Spülvorrichtung kann aus dem Situs anschließend von der Saugvorrichtung an der Spitze der Kanüleneinheit 5 abgesaugt werden.

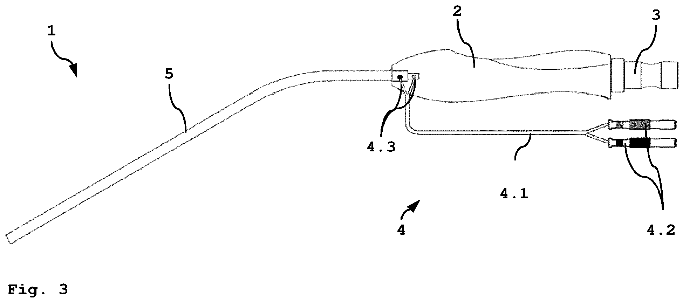

In Fig. 3 ist eine weitere Ausführungsform des Sauginstruments 1 gemäß dem ersten Aspekt der vorliegenden Erfindung schematisch dargestellt. Das Sauginstrument 1 aus Fig. 3 entspricht weitestgehend dem Sauginstrument 1 aus Fig. 1. Daher werden nur die Unterschiede nachfolgend erläutert.

Anstelle zweier Anschlusspins ist die zweite Schnittstelle 4 hier als bipolares Kabel 4.1 ausgeführt. Das bipolare Kabel 4.1 weist an seinem freien Ende zwei Buchsen 4.2 oder alternativ zwei Stecker auf, die direkt an ein IOM-System, genauer an die Stimulationseinrichtung angeschlossen werden können. Das bipolare Kabel 4.1 ist fest in das Handstück 2 integriert und mit zwei elektrischen Kontakten 4.3 (z. B. Lötstelle oder Schweißstelle) an dem äußeren Kanülenrohr 5.1 bzw. dem inneren Kanülenrohr 5.2 elektrisch angeschlossen.

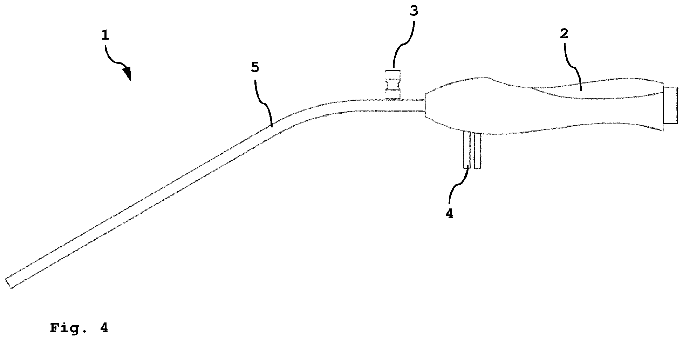

In Fig. 4 ist eine weitere Ausführungsform des Sauginstruments 1 gemäß dem ersten Aspekt der vorliegenden Erfindung schematisch dargestellt. Das Sauginstrument 1 aus Fig. 4 entspricht weitestgehend dem Sauginstrument 1 aus Fig. 1 und Fig.3. Daher werden nur die Unterschiede nachfolgend erläutert.

Die erste Schnittstelle 3 und die zweite Schnittstelle 4 sind hier anstelle an dem Handstück 2 direkt an der Kanüleneinheit 5 angeordnet bzw. mit dieser einstückig/integral ausgeformt. Die zweite Schnittstelle 4 kann entweder wie dargestellt durch zwei Anschlusspins oder alternativ durch ein bipolares Kabel 4.1, wie zu der Ausführungsform aus Fig. 3 beschrieben, realisiert werden. Es kann auch jeweils nur eine der beiden Schnittstellen 3, 4 direkt an der Kanüleneinheit 5 und die andere der beiden Schnittstellen 3, 4 entsprechend den Ausführungsbeispielen aus Fig. 1 und Fig. 3 an dem Handstück 2 angeordnet sein.

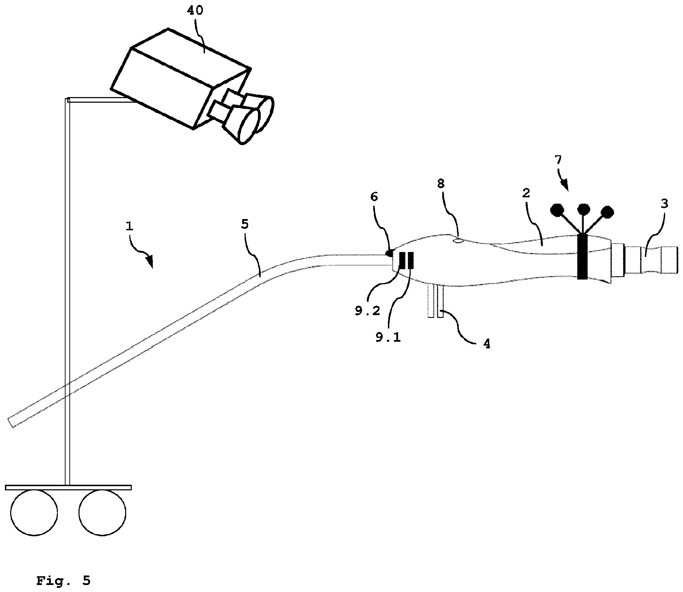

In Fig. 5 ist eine weitere Ausführungsform des Sauginstruments 1 gemäß dem ersten Aspekt der vorliegenden Erfindung schematisch dargestellt. Das Sauginstrument 1 aus Fig. 5 entspricht weitestgehend dem Sauginstrument 1 aus Fig. 1, Fig. 3 und Fig.4. Daher werden nur die Unterschiede nachfolgend erläutert.

Das Sauginstrument 1 umfasst hier ferner eine LED 6, ein Trackingelement 7, eine Saugkontrollöffnung 8, ein erstes Bedienelement 9.1 und ein zweites Bedienelement 9.2.

Die LED 6 ist in das Handstück 2 integriert. Die LED 6 kann entweder direkt an dem Handstück 2 oder über die Stimulationseinrichtung ein- und ausgeschaltet werden. Die LED 6 kann alternativ mit einem Lichtleiter (nicht dargestellt) verbunden werden, welcher sich entlang der äußeren Rohroberfläche des äußeren Kanülenrohrs 5.1 erstreckt, wo es den Situs ausleuchten kann. Die Lichtleiter leiten das von einer Lichtquelle bzw. von der LED abgestrahlte Licht weiter und beleuchten den Situs.

Das Trackingelement 7 ist hier an dem proximalen Ende des Handstücks 2 angeordnet. Alternativ kann es auch an dem proximalen Ende der Kanüleneinheit 5 angeordnet sein. Das Trackingelement 7 weist hier beispielhaft drei Marker in Form von reflektiven Kugeln auf, die in einem vorbestimmten ungleichseitigen Dreieck zueinander angeordnet sind. Die Lage und Pose des Trackingelements 7 kann von einer externen Navigationsvorrichtung 40 mit Stereokamera durch Triangulation ermittelt werden. Daraus lässt sich die Lage und Pose des Sauginstruments 1 und insbesondere der Spitze der Kanüleneinheit 5 in registrierten Bilddaten des Patienten darstellen.

Die Saugkontrollöffnung 8 ist an einer Oberseite des Handstücks 2 angeordnet und durch Fluidkanäle in dem Handstück 2 fluidleitend mit dem Lumen 5.4 des inneren Kanülenrohrs 5.2 verbunden. Die Saugöffnung 8 ist mit einem Daumen verschließbar, wodurch sich die Saugwirkung an der Spitze der Kanüleneinheit 5 kontrollieren lässt.

Das erste Bedienelement 9.1 ist als Schalter ausgeführt und an dem Handstück 2 oder alternativ an der Saugvorrichtung oder Spülvorrichtung (nicht dargestellt) oder alternativ am fluidleitenden Kabel oder alternativ an der Kanüleneinheit 5 angeordnet. Durch das erste Bedienelement 9.1 kann beispielsweise ein erstes steuerbares Ventil (nicht dargestellt) sowie ein zweites steuerbares Ventil (nicht dargestellt), die in dem Handstück 2 bevorzugt direkt an der ersten Schnittstelle 3 angeordnet sein können, gegengleich geöffnet und geschlossen werden. Das erste steuerbare Ventil kann das Lumen 5.4 des inneren Kanülenrohrs 5.2 fluidleitend mit der ersten Schnittstelle 3 und darüber mit der Saugvorrichtung verbinden. Das zweite steuerbare Ventil kann das Lumen 5.4 des inneren Kanülenrohrs 5.2 fluidleitend mit der ersten Schnittstelle 3 und darüber mit der Spülvorrichtung verbinden. Ist das erste steuerbare Ventil über das erste Bedienelement 9.1 in einer ersten Stellung geöffnet und das zweite steuerbare Ventil gleichzeitig geschlossen, so kann über die Saugvorrichtung ein Unterdruck an der Spitze der Kanüleneinheit 5 angelegt und Flüssigkeit und Gewebe aus dem Situs abgesaugt werden. Ist das erste steuerbare Ventil über das erste Bedienelement 9.1 in einer zweiten Stellung geschlossen und das zweite steuerbare Ventil gleichzeitig geöffnet, so kann über die Spülvorrichtung das Spülmedium an die Spitze der Kanüleneinheit 5 gefördert und der Situs gespült bzw. befeuchtet werden. Optional kann das Bedienelement 9.1 auch in eine dritte Stellung bewegbar sein, in der beide steuerbaren Ventile geschlossen sind.

Das zweite Bedienelement 9.2 ist als Schalter ausgeführt und an dem Handstück 2 angeordnet. Durch das zweite Bedienelement 9.2 wird zwischen einem monopolaren Betrieb und einem bipolaren Betrieb umgeschaltet. Dazu ist das zweite Bedienelement 9.2 kommunikativ über die zweite Schnittstelle 4 mit der Stimulationseinrichtung verbunden. In einer ersten Stellung des zweiten Bedienelements 9.2 wird die Steuerungseinrichtung veranlasst im bipolaren Betrieb zu arbeiten und Gewebe über beide Kanülenrohre 5.2 und 5.1 zu stimulieren, wobei die Reizantwort des stimulierten Gewebes über entsprechende Ableitelektroden am Patienten (nicht dargestellt) detektiert werden. In einer zweiten Stellung des zweiten Bedienelements 9.2 wird die Stimulationseinrichtung veranlasst im monopolaren Betrieb zu arbeiten und Gewebe durch beispielsweise das innere Kanülenrohr 5.2 elektrisch zu stimulieren, wobei ferner die Anbringung einer Gegenelektrode am Patienten (nicht dargestellt) notwendig ist. Über mindestens eine, vorzugsweise zwei Ableitelektroden am Patienten (nicht dargestellt) kann sodann die Reizantwort des stimulierten Gewebes detektiert werden. In einer dritten Stellung des zweiten Bedienelements 9.2 erfolgt keine Stimulation und ausschließlich ein separater Spül- oder Saugvorgang kann ausgeführt werden.

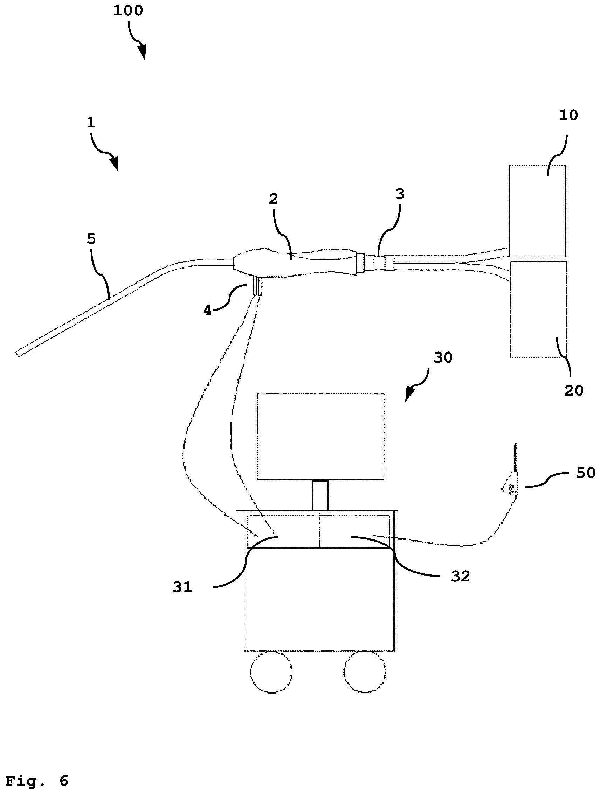

In Fig. 6 ist das System 100 zum Absaugen von Flüssigkeiten und Gewebe sowie zum Überwachen von Nervengewebe gemäß dem zweiten Aspekt der vorliegenden Erfindung schematisch dargestellt. Das System 100 umfasst eines der Sauginstrumente 1 aus den Figs. 1 bis 5 gemäß dem ersten Aspekt der vorliegenden Erfindung sowie die Saugvorrichtung 10, die Spülvorrichtung 20, ein IOM-System 30 und mindestens eine, vorzugsweise zwei zusätzliche Ableitelektroden 50 (der Übersicht halber ist nur eine Ableitelektrode dargestellt).

Das IOM-System 30 kann eine Steuervorrichtung umfassend eine Datenverarbeitungseinrichtung (z. B. Computer, Laptop usw.) sein. Das IOM-System 30 umfasst eine Stimulationseinrichtung 31 und eine Überwachungseinrichtung 32, die mit der Stimulationseinrichtung 31 kommunikativ verbunden ist. Die Stimulationseinrichtung 31 und die Überwachungseinrichtung 32 können als separate Hardwaremodule in das IOM-System 30 integriert sein oder als Softwaremodule im IOM-System 30 implementiert sein.

Das Sauginstrument 1 ist an der ersten Schnittstelle 3 über einen semirigiden Schlauch, der beispielhaft zwei separate Fluidleitungen umfasst, sowohl mit der Saugvorrichtung 10 als auch mit der Spülvorrichtung 20 fluidleitend verbunden. Weiter ist das Sauginstrument 1 an der zweiten Schnittstelle 4 über ein bipolares Kabel mit dem IOM-System 30 bzw. direkt mit der Stimulationseinrichtung 31 elektrisch verbunden.

Ist der bipolare Betrieb ausgewählt (beispielsweise über das zweite Bedienelement, hier nicht dargestellt), so kann die Stimulationseinrichtung 31 Gewebe, das mit der Spitze der Kanüleneinheit 5 berührt wird, über beide Kanülenrohre 5.1 und 5.2 elektrisch stimulieren. Die Reizantwort des stimulierten Gewebes wird über eine, vorzugsweise zwei zusätzlich am Patienten angebrachten Ableitelektroden 50 (hier ausgeführt als Nadelelektrode) detektiert und als Ableitsignal an die Überwachungseinrichtung 32 weitergegeben. Das Ableitsignal kann von einem Verstärker (analog oder digital, nicht dargestellt) verstärkt und/oder von einer Filtereinrichtung (nicht dargestellt) gefiltert werden. Der Anwender, beispielsweise ein Chirurg, und alternativ oder zusätzlich die Überwachungseinrichtung 32 überwachen das Ableitsignal und vergleichen dieses mit vordefinierten Grenzwerten. Die Überwachungseinrichtung 32 kann das Detektionssignal und zusätzlich ein Warnsignal, falls ein Grenzwert überschritten wird, visuell über einen Bildschirm (nicht dargestellt) und/oder akustisch über einen Lautsprecher (nicht dargestellt) ausgeben.

Die Ableitelektroden 50, hier Nadelelektroden oder alternativ Oberflächenelektroden, sind mit dem IOM-System 30 oder direkt mit der Überwachungseinrichtung 32 über Kabel elektrisch verbunden.

Sofern die Stimulationseinrichtung 31 auf den monopolaren Betrieb eingestellt ist, wird beispielsweise über das innere Kanülenrohr 5.2 und eine zusätzlich am Patienten angebrachte und mit der Stimulationseinrichtung 31 bzw. dem IOM-System 30 elektrisch verbundene Gegenelektrode (nicht dargestellt) Gewebe im Situs stimuliert und die Reizantwort des stimulierten Gewebes über die Ableitelektroden 50 detektiert. Die detektierte Reizantwort wird als Ableitsignal an die Überwachungseinrichtung 32 weitergegeben. Das Ableitsignal kann von einem Verstärker (analog oder digital, nicht dargestellt) verstärkt und zusätzlich oder alternativ von einer Filtereinrichtung (nicht dargestellt) gefiltert werden. Der Anwender, beispielsweise ein Chirurg, und alternativ oder zusätzlich die Überwachungseinheit 32 überwachen das Ableitsignal und vergleichen dieses mit vordefinierten Grenzwerten. Die Überwachungseinheit 32 kann das Ableitsignal und ein Warnsignal, falls ein Grenzwert überschritten wird, visuell über einen Bildschirm (nicht dargestellt) und/oder akustisch über einen Lautsprecher (nicht dargestellt) ausgeben.

Vor der Durchführung beispielsweise einer Tumorresektion im Gehirn wird das Sauginstrument 1 über das bipolare Kabel mit dem IOM-System 30 oder direkt mit der Stimulationseinrichtung 31 verbunden. Die Ableitung erfolgt über zusätzlich eingebrachten Ableitelektroden 50 in (Nadelelektrode) oder an (Oberflächenelektrode) Muskeln. Hierbei werden vorzugsweise verschiedene Muskeln der oberen Extremitäten oder des Kopfes ausgewählt und mit mehreren Ableitelektroden 50 versehen. Während der Tumorresektion im Gehirn wird die Spitze der Kanüleneinheit 5 an der zu resezierenden Stelle platziert und durch Stromzugabe auf das innere Kanülenrohr 5.2 das Gewebe an der Spitze der Kanüleneinheit 5 zwischen dem inneren Kanülenrohr 5.2 und dem äußeren Kanülenrohr 5.1 elektrisch stimuliert. Es wird überprüft, ob die Stimulation an dieser Stelle ein MEP (motorisch evoziertes Potential) auslöst oder nicht. Wird kein MEP auf dem motorischen Kortex ausgelöst, handelt es sich um tumoröses Gewebe, das reseziert werden kann, wohingegen eine erfolgreiche MEP-Ableitung anzeigt, dass es sich um funktionell relevantes Gewebe handelt und die Stimulation und Resektion an einer anderen Stelle fortgeführt werden sollte. Die monopolare Stimulation erfolgt über einen der beiden Stimulationspole an der Kanüleneinheit 5, vorzugsweise das inneren Kanülenrohr 5.2, und einer zusätzlich am Patienten eingebrachten Gegenelektrode. Die Ableitelektroden 50 werden gemäß der bipolaren Stimulation platziert. Im vorliegenden Fall kann die monopolare Stimulation auch als Abstandsradar dienen. Abhängig von der Stimulationsintensität kann abgeschätzt werden, in welchem Abstand sich die motorischen Bahnen befinden. Hier gilt eine Faustregel von 1 mA pro 1 mm. Das bedeutet, dass eine erfolgreiche MEP-Stimulation mit 5 mA anzeigt, dass sich die Pyramidenbahn etwa in 5 mm Entfernung befindet. Die Spitze der Kanüleneinheit 5 kann sich während kritischer Phasen des Eingriffs zu jeder Zeit am zu untersuchendem Gewebe befinden und dieses kontinuierlich stimulieren (kontinuierliches subkortikales Mapping). Durch das Lumen 5.4 des inneren Kanülenrohrs 5.2 werden Flüssigkeiten und Tumorgewebe abgesaugt, ohne die in der Nähe befindlichen Nervenbahnen des Patienten zu schädigen. Schwerwiegende Folgen wie Ausfallserscheinungen und Lähmungen werden zum größten Teil vermieden.

Das nachfolgende Beispiel dient der Illustration der vorliegenden Erfindung. Thematisiert wird die Entfernung eines Vestibularisschwannoms, welches auch Akustikusneurinom (AKN) genannt wird. Das Vestibularisschwannom ist ein Tumor, welcher von der Hüllschicht des Nervus vestibulocochlearis ausgeht und diesem Nerv direkt anliegt. Der N. vestibulocochlearis ist ein Hirnnerv, welcher für die Hör- und Gleichgewichtsfunktion zuständig ist. Klassische Beschwerden von Patienten mit Vestibularisschwannom sind Schwindel, Hörminderung und Tinnitus. Ausgeprägte Vestibularisschwannome können außerdem die Funktion des Gesichtsnervs (N. facialis) beeinträchtigen. Die Entfernung eines AKNs erfolgt in der Neurochirurgie unter dem Einsatz von intraoperativem Neuromonitoring. Essenziell hierbei ist die Überwachung der Hörfunktion mittels akustisch evozierter Potentiale und der Funktion des N. facialis mittels elektromyografischer (EMG) Messung. Hierbei kommt auch die elektrische Stimulation zum Einsatz. Bei ausgedehnten Tumoren kann die Überwachung weiterer Hirnnerven (z.B. des N. trigeminus und der kaudalen Hirnnerven) mittels EMG angeraten sein.

Der bipolare Mappingsauger wird in diesem Fall für die direkte Stimulation des N. facialis oder der weiteren Hirnnerven eingesetzt. Hierbei können sowohl die bipolare als auch die monopolare Stimulation verwendet werden, was bedeutet, dass die Ableitelektroden 50 in der Nähe des Situs platziert werden. Die zwei Anschlusspins der zweiten Schnittstelle 4 des Sauginstruments 1 werden über ein bipolares Kabel mit Touch-Proof-Buchsen mit der Stimulationseinrichtung 31 des IOM-Systems 30 verbunden. Das Handstück 2 des Sauginstruments 1 wird mit einem semirigiden Schlauch an der ersten Schnittstelle 3 fluidleitend mit einer Saugvorrichtung 10 verbunden. Die Ableitelektroden 50 werden in diesem Fall in der Zielmuskulatur der Hirnnerven platziert. Beim N. facialis beispielweise ist dies vorwiegend die mimische Muskulatur des Gesichts.

Nachdem der Zugang durch Hautschnitt und Kraniotomie hinter dem Ohr erfolgt ist, kann mittels monopolarer Stimulation, welche entweder durch das zweite Bedienelement 9.2 (Schalter) am Handstück 2 oder Diskonnektierung einer der Buchsen des bipolaren Kabels aktiviert wird, die generelle Funktion des N. facialis, sowie eine ungefähre Abstandsbestimmung zum Nerv durch Stimulationsschwellwertbestimmung abgeschätzt werden. Während der Stimulation wird auf einem Monitor des IOM-Systems 30 die Reizantwort in Echtzeit zur Interpretation angezeigt und akustisch über einen Lautsprecher kenntlich gemacht. Gleichzeitig kann das Absaugen von Flüssigkeiten oder Gewebe mit dem gleichen Instrument erfolgen. Im weiteren Verlauf der Operation, wenn diese in direkter Nähe zu den Hirnnerven erfolgt, wird auf die bipolare Funktion umgestellt (durch das zweite Bedienelement 9.2 (Schalter) am Handstück 2 oder Konnektierung der zweiten Buchse des bipolaren Kabels an der zweiten Schnittstelle 4). Nun können die einzelnen Hirnnerven durch bipolare Stimulation sehr selektiv identifiziert werden. Durch das kontinuierliche Mapping weiß der Chirurg genau, wo sich die Hirnnerven und der Tumor befinden und kann so die Nerven besser und gezielt schonen. Sobald eine Reizantwort ausgelöst wird, handelt es sich um gesundes und funktionsfähiges Nervengewebe. Wird hingegen keine Reizantwort ausgelöst, handelt es sich um tumoröses Gewebe, welches reseziert werden soll. Dadurch, dass der Sauger und die Stimulationssonde in dem bipolaren Mappingsauger vereint sind, ist eine engmaschige Überwachung der Nerven und Kontrolle deren Funktion möglich, da kein Instrumentenwechsel erfolgen muss. Zusätzlich oder alternativ kann die Impedanzspektroskopie verwendet werden, um mittels Messung der Impedanz von dem an der Spitze des Kanülenrohrs 5 umliegenden Gewebe eine Differenzierung von Tumorgewebe und gesundem Gewebe zu erreichen.

Um ein Austrocknen des Situs oder ein Antrocknen von Operationsrückständen zu vermeiden, kann mit dem Sauginstrument 1 der Situs auch durchgespült werden. Die Spülflüssigkeiten sowie die Gewebereste können anschließend mit dem Sauginstrument 1 abgesaugt werden. Der Absaugvorgang bzw. die Saugstärke kann mit der Saugkontrollöffnung 8 am Handstück 2 gesteuert werden.

Aus dem Handstück 2 oder alternativ aus der Spitze der Kanüleneinheit 5 wird Licht über eine LED bzw. einen Lichtleiter ausgestrahlt. Dadurch wird der Operationsbereich zusätzlich beleuchtet. Optional kann zusätzlich das Trackingelement 7 auf das Handstück 2 gesteckt werden. Kameras des Navigationssystems 40 können dann die aktuelle Lage und Pose des Sauginstruments 1 ermitteln und diese verfolgen. Dies bietet vor allem in der Neurochirurgie und Spinalchirurgie wesentliche Vorteile, wie zum Beispiel eine erhöhte Präzision und Orientierung bei minimalinvasiven chirurgischen Eingriffen.

Der wesentliche Vorteil des bipolaren Mappingsaugers ist somit der, dass ein umständliches Wechseln der Instrumente nicht mehr nötig ist. Das Sauginstrument 1 vereint einen Sauger mit zwei Stimulationskontakten. Das Ziel einer vollständigen Tumorresektion, ohne in der Nähe befindliche Nervenbahnen zu schädigen, wird mit dem Sauginstrument 1 unterstützt und vereinfacht. Ferner wird durch die Vermeidung des Instrumentenwechsels die Operationszeit verkürzt. Durch die bipolare Stimulation ist auch eine fokussierte und selektive Stimulation und Identifikation von Nervenbahnen möglich, wobei die Umschaltfunktion zwischen dem bipolaren Betrieb und dem monopolaren Betrieb die Verschmelzung beider Methoden in einem Instrument ermöglicht.

Obwohl die vorliegende Erfindung anhand bevorzugter Ausführungsbeispiele vorstehend vollständig beschrieben wurde, ist sie darauf nicht beschränkt, sondern auf viel-fältige Art und Weise modifizierbar.

### Bezugszeichenliste

- 1: Sauginstrument
- 2: Handstück
- 3: erste Schnittstelle
- 4: zweite Schnittstelle
- 4.1: bipolares Kabel
- 4.2: Buchsen
- 4.3: elektrischer Kontakt
- 5: Kanüleneinheit
- 5.1: äußeres Kanülenrohr
- 5.2: inneres Kanülenrohr
- 5.3: Isolation
- 6: LED
- 7: Trackingelement
- 8: Saugkontrollöffnung
- 9.1: erstes Bedienelement
- 9.2: zweites Bedienelement
- 10: Saugvorrichtung
- 20: Spülvorrichtung
- 30: IOM-System
- 31: Stimulationseinrichtung
- 32: Überwachungseinrichtung
- 40: Navigationsvorrichtung
- 50: Ableitelektrode

## Patentansprüche

1. Bipolarer Mappingsauger (1), für chirurgische Zwecke, umfassend:
- ein Handstück (2);
- eine erste Schnittstelle (3), die ausgebildet ist, zumindest eine Fluidverbindung mit einer externen Saugvorrichtung (10) und/oder Spülvorrichtung (20) herzustellen;
- eine zweite Schnittstelle (4), die ausgebildet ist, eine bipolare elektrische Verbindung mit einer externen Stimulationseinrichtung (31) herzustellen; und
- einer Kanüleneinheit (5), die sich von dem Handstück (2) aus in eine axiale Richtung erstreckt und an einem proximalen Ende der Kanüleneinheit (5) mit dem Handstück (2) mechanisch verbunden ist, umfassend:
- ein elektrisch leitfähiges inneres Kanülenrohr (5.2), das mit einem ersten Pol der bipolaren elektrischen Verbindung der zweiten Schnittstelle (4) elektrisch verbunden ist, konzentrisch in einem äußeren Kanülenrohr (5.1) angeordnet ist und mit der ersten Schnittstelle (3) fluidleitend verbunden ist;
- ein elektrisch leitfähiges äußeres Kanülenrohr (5.1), das mit einem zweiten Pol der bipolaren elektrischen Verbindung der zweiten Schnittstelle (4) elektrisch verbunden ist; und
- eine Isolation (5.3), die konzentrisch zwischen dem äußeren Kanülenrohr (5.1) und dem inneren Kanülenrohr (5.2) angeordnet und ausgebildet ist, das äußere Kanülenrohr (5.1) und das innere Kanülenrohr (5.2) vollständig gegeneinander elektrisch zu isolieren.

2. Bipolarer Mappingsauger (1) gemäß Anspruch 1, ferner umfassend:
- eine Beleuchtungseinrichtung, die einen Lichtaustritt, eine Lichtquelle (6)und/oder einen Lichtleiter umfasst,
wobei der Lichtaustritt an dem Handstück (2) oder alternativ an der Kanüleneinheit (5) angeordnet und ausgebildet ist, Licht von dem Lichtleiter oder alternativ von der Lichtquelle (6) in Richtung eines Situs austreten zu lassen,
wobei der Lichtleiter ausgebildet ist, Licht, das von der Lichtquelle (6) oder alternativ von einer externen Lichtquelle ausgesendet wird, zu dem Lichtaustritt zu leiten.

3. Bipolarer Mappingsauger (1) gemäß einem der vorherigen Ansprüche, ferner umfassend ein Trackingelement (7), das eingerichtet ist, von einer externen Navigationsvorrichtung (40) erkannt zu werden, und fest oder lösbar mechanisch mit dem Handstück (2) oder der Kanüleneinheit (5) verbunden ist.

4. Bipolarer Mappingsauger (1) gemäß einem der vorherigen Ansprüche, wobei das äußere Kanülenrohr (5.1) und optional das innere Kanülenrohr (5.2) und/oder die Isolation (5.3) biokompatibel oder bioinert ausgeführt sind und insbesondere wobei das äußere Kanülenrohr (5.1) und/oder das innere Kanülenrohr (5.2) aus Edelstahl und/oder die Isolation (5.3) aus Kunststoff, insbesondere Polyamid, gefertigt sind.

5. Bipolarer Mappingsauger (1) gemäß einem der vorherigen Ansprüche, wobei die Kanüleneinheit (5) einen äußeren Durchmesser von 1 mm [Millimeter] bis 15 mm aufweist.

6. Bipolarer Mappingsauger (1) gemäß einem der vorherigen Ansprüche, wobei die Kanüleneinheit (5) eine Länge von 10 cm [Zentimeter] bis 40 cm aufweist.

7. Bipolarer Mappingsauger (1) gemäß einem der vorherigen Ansprüche, wobei die Kanüleneinheit (5) gerade ausgeführt ist oder alternativ in einem proximalen Bereich eine Abwinklung mit einem Winkel von 10° [Grad] bis 60°, bevorzugt von 30° aufweist.

8. Bipolarer Mappingsauger (1) gemäß einem der vorherigen Ansprüche, wobei das Handstück (2) eine Saugkontrollöffnung (8) umfasst, die mit dem inneren Kanülenrohr (5.2) fluidleitend verbunden ist.

9. Bipolarer Mappingsauger (1) gemäß einem der vorherigen Ansprüche, wobei die Kanüleneinheit (5) an dem proximalen Ende der Kanüleneinheit (5) fest oder lösbar mechanisch mit dem Handstück (2) verbunden ist.

10. Bipolarer Mappingsauger (1) gemäß einem der vorherigen Ansprüche, ferner umfassend:
- ein erstes steuerbares Ventil, wobei das innere Kanülenrohr (5.2) über das erste steuerbare Ventil und einen ersten Fluidanschluss der ersten Schnittstelle (3) mit der externen Saugvorrichtung (10) fluidleitend verbunden ist, und/oder
- ein zweites steuerbares Ventil, wobei das innere Kanülenrohr (5.2) über das zweite steuerbare Ventil und einen zweiten Fluidanschluss der ersten Schnittstelle (3) mit der externen Spülvorrichtung (20) fluidleitend verbunden ist.

11. Bipolarer Mappingsauger (1) gemäß Anspruch 10, das Handstück (2) ferner umfassend ein erstes Bedienelement (9.1) das ausgebildet ist, zwischen einer Saugfunktion der externen Saugvorrichtung (10) und einer Spülfunktion der externen Spülvorrichtung (20) umzuschalten.

12. Bipolarer Mappingsauger (1) gemäß einem der vorherigen Ansprüche, wobei die zweite Schnittstelle (4) zwei Anschlusspins zur Herstellung einer monopolaren oder bipolaren Verbindung umfasst oder wobei die zweite Schnittstelle (4) ein bipolares Kabel zur Herstellung der monopolaren oder bipolaren elektrischen Verbindung umfasst.

13. Bipolarer Mappingsauger (1) gemäß einem der vorherigen Ansprüche, das Handstück (2) ferner umfassend ein zweites Bedienelement (9.2), wobei die zweite Schnittstelle (4) ferner ausgebildet ist, eine kommunikative Verbindung des zweiten Bedienelements mit der externen Stimulationseinrichtung (31) herzustellen, und wobei das zweite Bedienelement (9.2) ausgebildet ist, zwischen einem monopolaren Betrieb und einem bipolaren Betrieb umzuschalten.

14. System (100) zum Absaugen von Flüssigkeiten und Gewebe und/oder Spülen des Situs sowie zum Überwachen von Nervengewebe, umfassend:
- den Mappingsauger gemäß einem der vorherigen Ansprüche;
- eine Saugvorrichtung (10) und/oder eine Spülvorrichtung (20);
- mindestens eine, vorzugsweise zwei Ableitelektroden (50), die an einem Patienten in der Nähe zu überwachenden Nervengewebes anbringbar sind und
- ein IOM-System (30), das eine Stimulationseinrichtung (31) und eine Überwachungseinrichtung (32), die mit der Stimulationseinrichtung (31) kommunikativ verbunden ist, umfasst,
wobei die Saugvorrichtung (10) und/oder die Spülvorrichtung (20) mittels einer Fluidleitung (11, 12), insbesondere eines semirigiden Schlauchs, über die erste Schnittstelle (3) mit dem Sauginstrument (1) fluidleitend verbunden ist,
wobei die Stimulationseinrichtung (31) des IOM-Systems (30) mittels einer monopolaren oder bipolaren Verbindung (33), insbesondere eines monopolaren oder bipolaren Kabels, über die zweite Schnittstelle (4) mit dem Sauginstrument (1) monopolar oder bipolar elektrisch verbunden ist,
wobei die Stimulationseinrichtung (31) ausgebildet ist, Gewebe über mindestens eines der beiden Kanülenrohre (5.1, 5.2) und/oder über beide Kanülenrohre (5.1, 5.2) elektrisch zu stimulieren, und
wobei die Überwachungseinrichtung (32) mit den mindestens zwei Ableitelektroden (50) elektrisch verbunden ist und ausgebildet ist, eine von den mindestens zwei Ableitelektroden (50) aufgezeichnete Reizantwort zu überwachen und/oder an einen Anwender akustisch und/oder visuell auszugeben.

15. System (100) gemäß Anspruch 14, wobei das System (100) entweder ferner ein Impedanzmessgerät umfasst, das mit der Überwachungseinheit kommunikativ verbunden und ausgebildet ist, eine Impedanzmessung durchzuführen, oder alternativ wobei das IOM-System (30) ausgebildet ist, die Impedanzmessung durchzuführen, wobei zur Impedanzmessung komplexe Widerstände bei verschiedenen Frequenzen von an der Spitze der Kanüleneinheit (5) umliegendem Gewebe gemessen werden und wobei das System ausgebildet ist, basierend auf den gemessenen Impedanzen die Art des Gewebes zu bestimmen und an den Nutzer auszugeben.

## Claims

1. Bipolar mapping suction device (1) for surgical purposes, comprising:
- a handpiece (2);
- a first interface (3) configured to establish at least one fluid connection with an external suction device (10) and/or rinsing device (20);
- a second interface (4) configured to establish a bipolar electrical connection with an external stimulation device (31); and
- a cannula unit (5) extending from the handpiece (2) in an axial direction and mechanically connected to the handpiece (2) at a proximal end of the cannula unit (5), the cannula unit comprising:
- an electrically conductive inner cannula tube (5.2) which is electrically connected to a first pole of the bipolar electrical connection of the second interface (4), is concentrically arranged in an outer cannula tube (5.1) and is connected to the first interface (3) in a fluid-conducting manner;
- an electrically conductive outer cannula tube (5.1) electrically connected to a second pole of the bipolar electrical connection of the second interface (4); and
- insulation (5.3) concentrically arranged between the outer cannula tube (5.1) and the inner cannula tube (5.2) and configured to fully electrically insulate the outer cannula tube (5.1) and the inner cannula tube (5,2) in relation to one another.

2. Bipolar mapping suction device (1) as claimed in claim 1, further comprising:
- an illumination device comprising a light outlet, a light source (6) and/or a light guide,
wherein the light outlet is arranged on the handpiece (2) or alternatively on the cannula unit (5) and is configured to allow light to exit from the light guide or alternatively from the light source (6) in the direction of a situs,
wherein the light guide is configured to guide light emitted from the light source (6) or alternatively from an external light source to the light outlet.

3. Bipolar mapping suction device (1) as claimed in any one of the preceding claims, further comprising a tracking element (7) configured to be detected by an external navigation device (40) and fixedly or detachably mechanically connected to the handpiece (2) or the cannula unit (5).

4. Bipolar mapping suction device (1) as claimed in any one of the preceding claims, wherein the outer cannula tube (5.1) and optionally the inner cannula tube (5.2) and/or the insulation (5.3) are biocompatible or bioinert and in particular wherein the outer cannula tube (5.1) and/or the inner cannula tube (5.2) are made of stainless steel and/or the insulation (5.3) is made of synthetic material, in particular polyamide.

5. Bipolar mapping suction device (1) as claimed in any one of the preceding claims, wherein the cannula unit (5) has an outer diameter of 1 mm [millimetre] to 15 mm.

6. Bipolar mapping suction device (1) as claimed in any one of the preceding claims, wherein the cannula unit (5) has a length of 10 cm [centimetres] to 40 cm.

7. Bipolar mapping suction device (1) as claimed in any one of the preceding claims, wherein the cannula unit (5) is straight or alternatively has a bend with an angle of 10° [degrees] to 60°, preferably of 30°, in a proximal region.

8. Bipolar mapping suction device (1) as claimed in any one of the preceding claims, wherein the handpiece (2) comprises a suction control opening (8) which is connected to the inner cannula tube (5.2) in a fluid-conducting manner.

9. Bipolar mapping suction device (1) as claimed in any one of the preceding claims, wherein the cannula unit (5) is fixedly or detachably mechanically connected to the handpiece (2) at the proximal end of the cannula unit (5).

10. Bipolar mapping suction device (1) as claimed in any one of the preceding claims, further comprising:
- a first controllable valve, wherein the inner cannula tube (5.2) is connected in a fluid-conducting manner to the external suction device (10) via the first controllable valve and a first fluid connection of the first interface (3), and/or
- a second controllable valve, wherein the inner cannula tube (5.2) is connected in a fluid-conducting manner to the external rinsing device (20) via the second controllable valve and a second fluid connection of the first interface (3) .

11. Bipolar mapping suction device (1) as claimed in claim 10, the handpiece (2) further comprising a first operating element (9.1) which is configured to switch between a suction function of the external suction device (10) and a rinsing function of the external rinsing device (20).

12. Bipolar mapping suction device (1) as claimed in any one of the preceding claims, wherein the second interface (4) comprises two connection pins for establishing a monopolar or bipolar connection, or wherein the second interface (4) comprises a bipolar cable for establishing the monopolar or bipolar electrical connection.

13. Bipolar mapping suction device (1) as claimed in any one of the preceding claims, the handpiece (2) further comprising a second operating element (9.2), wherein the second interface (4) is further configured to establish a communicative connection of the second operating element with the external stimulation device (31), and wherein the second operating element (9.2) is configured to switch between a monopolar operation and a bipolar operation.

14. System (100) for suctioning fluids and tissue and/or flushing the situs as well as for monitoring nerve tissue, comprising:
- the mapping suction device as claimed in any one of the preceding claims;
- a suction device (10) and/or a rinsing device (20);
- at least one, preferably two, conductive electrodes (50) which can be attached to a patient in the vicinity of nerve tissue to be monitored and
- an IOM system (30) comprising a stimulation device (31) and a monitoring device (32) communicatively connected to the stimulation device (31),
wherein the suction device (10) and/or the rinsing device (20) is connected in a fluid-conducting manner to the suction instrument (1) by means of a fluid line (11, 12), in particular a semirigid tube, via the first interface (3),
wherein the stimulation device (31) of the IOM system (30) is electrically connected to the suction instrument (1) in a monopolar or bipolar manner by means of a monopolar or bipolar connection (33), in particular a monopolar or bipolar cable, via the second interface (4),
wherein the stimulation device (31) is configured to electrically stimulate tissue via at least one of the two cannula tubes (5.1, 5.2) and/or via both cannula tubes (5.1, 5.2), and
wherein the monitoring device (32) is electrically connected to the at least two conductive electrodes (50) and is configured to monitor a stimulus response recorded by the at least two conductive electrodes (50) and/or to output same acoustically and/or visually to a user.

15. System (100) as claimed in claim 14, wherein the system (100) either further comprises an impedance measurement apparatus communicatively connected to the monitoring unit and configured to perform an impedance measurement, or alternatively wherein the IOM system (30) is configured to perform the impedance measurement, wherein complex resistances at different frequencies of tissue surrounding the tip of the cannula unit (5) are measured for impedance measurement, and wherein the system is configured to determine and output to the user the type of tissue based on the measured impedances.

## Revendications

1. Dispositif d'aspiration à mappage bipolaire (1),
à des fins chirurgicales, comprenant :
- une pièce à main (2) ;
- une première interface (3), qui est conçue pour établir au moins une liaison fluidique avec un dispositif d'aspiration extérieur (10) et/ou un dispositif de lavage (20) ;
- une deuxième interface (4), qui est conçue pour établir une connexion électrique bipolaire avec un dispositif de stimulation extérieur (31) ; et
- une unité de canule (5), qui s'étend à partir de la pièce à main (2) dans une direction axiale, et est reliée mécaniquement à une extrémité proximale de l'unité de canule (5) avec la pièce à main (2), comprenant :
- un tube de canule intérieur (5.2) conducteur électriquement, qui est relié électriquement avec un premier pôle de la connexion électrique bipolaire de la deuxième interface (4), est disposé de manière concentrique dans un tube de canule extérieur (5.1) et est relié de manière conductrice de fluide avec la première interface (3) ;
- un tube de canule extérieur (5.1) conducteur électriquement, qui est relié électriquement avec un deuxième pôle de connexion électrique bipolaire de la deuxième interface (4) ; et
- une isolation (5.3), qui est disposée de manière concentrique entre le tube de canule extérieur (5.1) et le tube de canule intérieur (5.2) et est conçue pour isoler électriquement et totalement le tube de canule extérieur (5.1) et le tube de canule intérieur (5.2) l'un par rapport à l'autre.

2. Dispositif d'aspiration à mappage bipolaire (1) selon la revendication 1, comprenant en outre :
- un dispositif d'éclairage qui comprend une sortie de lumière, une source de lumière (6) et/ou un guide de lumière,
dans lequel la sortie de lumière est disposée sur la pièce à main (2) ou alternativement sur l'unité de canule (5) et est conçue pour laisser sortir de la lumière du guide de lumière ou alternativement de la source de lumière (6) en direction d'un site,
dans lequel le guide de lumière est conçu pour diriger de la lumière qui est émise par la source de lumière (6) ou alternativement une source de lumière extérieure vers la sortie de lumière.

3. Dispositif d'aspiration à mappage bipolaire (1) selon l'une des revendications précédentes,
comprenant en outre un élément de suivi (7), qui est conçu pour être reconnu par un dispositif de navigation (40) extérieur, et est relié solidement ou mécaniquement de manière amovible avec la pièce à main (2) ou l'unité de canule (5).

4. Dispositif d'aspiration à mappage bipolaire (1) selon l'une des revendications précédentes,
dans lequel le tube de canule extérieur (5.1) et, éventuellement, le tube de canule intérieur (5.2), et/ou l'isolation (5.3), sont conçus biocompatibles ou inertes biologiquement et en particulier dans lequel le tube de canule extérieur (5.1), et/ou le tube de canule intérieur (5.2), sont fabriqués en acier inoxydable, et/ou l'isolation (5.3) est fabriquée en matière plastique, notamment en polyamide.

5. Dispositif d'aspiration à mappage bipolaire (1 selon l'une des revendications précédentes,
dans lequel l'unité de canule (5) présente un diamètre extérieur de 1 mm [millimètre] à 15 mm.

6. Dispositif d'aspiration à mappage bipolaire (1) selon l'une des revendications précédentes,
dans lequel l'unité de canule (5) présente une longueur de 10 cm [centimètres] à 40 cm.

7. Dispositif d'aspiration à mappage bipolaire (1) selon l'une des revendications précédentes,
dans lequel l'unité de canule (5) est conçue rectiligne ou alternativement présente un pliage dans une zone proximale avec un angle de 10° [degrés] à 60°, de préférence de 30°.

8. Dispositif d'aspiration à mappage bipolaire (1) selon l'une des revendications précédentes,
dans lequel la pièce à main (2) comprend un orifice de commande d'aspiration (8) qui est relié en conduisant du fluide avec le tube de canule intérieur (5.2).

9. Dispositif d'aspiration à mappage bipolaire (1) selon l'une des revendications précédentes,
dans lequel l'unité de canule (5) est reliée solidement ou est amovible mécaniquement à l'extrémité proximale de l'unité de canule (5) avec la pièce à main (2).

10. Dispositif d'aspiration à mappage bipolaire (1) selon l'une des revendications précédentes,
comprenant en outre :
- une première soupape réglable, le tube de canule intérieur (5.2) étant relié en conduisant les fluides par le biais de la première soupape pouvant être commandée et d'un premier raccordement de fluide de la première interface (3) avec le dispositif d'aspiration extérieur (10), et/ou
- une deuxième soupape réglable, le tube de canule intérieur (5.2) étant relié en conduisant les fluides par le biais de la deuxième soupape pouvant être commandée et d'un deuxième raccordement de fluide de la première interface (3) avec le dispositif de lavage (10) extérieur.

11. Dispositif d'aspiration à mappage bipolaire (1) selon la revendication 10, la pièce à main (2) comprenant en outre un premier élément d'actionnement (9.1) qui est conçu pour se commuter entre une fonction d'aspiration du dispositif d'aspiration extérieur (10) et une fonction de lavage du dispositif de lavage (20) extérieur.

12. Dispositif d'aspiration à mappage bipolaire (1) selon l'une des revendications précédentes,
dans lequel la deuxième interface (4) comprend deux broches de raccordement pour l'établissement d'une connexion mono polaire ou bipolaire, ou dans lequel la deuxième interface (4) comprend un câble bipolaire pour l'établissement de la connexion électrique mono polaire ou bipolaire.

13. Dispositif d'aspiration à mappage bipolaire (1) selon l'une des revendications précédentes,
la pièce à main (2) comprenant en outre un deuxième élément d'actionnement (9.2), dans lequel la deuxième interface (4) est en outre conçue pour établir une liaison communicative du deuxième élément d'actionnement avec le dispositif de stimulation (31) extérieur, et dans lequel le deuxième élément d'actionnement (9.2) est conçu pour commuter entre un fonctionnement mono polaire et un fonctionnement bipolaire.

14. Système (100) d'aspiration de liquides et de tissus, et/ou de lavage d'un site, ainsi que pour la surveillance de tissus nerveux, comprenant :
- le dispositif d'aspiration à mappage (1) selon l'une des revendications précédentes ;
- un dispositif d'aspiration (10) et/ou un dispositif de lavage (20) ;
- au moins une, de préférence deux électrodes de décharge (50), qui peuvent être apportées sur un patient à proximité d'un tissu nerveux à surveiller, et- un système de l'Académie Nationale de Médecine IOM (30), qui comprend un dispositif de stimulation (31) et un dispositif de surveillance (32) qui est relié de manière communicative avec le dispositif de stimulation (31),
dans lequel le dispositif d'aspiration (10), et/ou le dispositif de lavage (20), sont reliés en conduisant les fluides au moyen d'une conduite de fluides (11, 12), notamment un tuyau semi rigide, par le biais de la première interface (3) avec l'instrument d'aspiration (1),
dans lequel le dispositif de stimulation (31) du système IOM (30) est relié électriquement de manière mono polaire ou bipolaire au moyen d'une connexion mono polaire ou bipolaire (33), notamment d'un câble mono polaire ou bipolaire, par le biais de la deuxième interface (4) avec l'instrument d'aspiration (1),
dans lequel le dispositif de stimulation (31) est conçu pour stimuler électriquement le tissu par le biais d'au moins un des deux tubes de canule (5.1, 5.2) et/ou par le biais des deux tubes de canule (5.1, 5.2), et
dans lequel le dispositif de surveillance (32) est relié électriquement avec les au moins deux électrodes de décharge (50) et est conçu pour surveiller une réponse d'excitation représentée par une des au moins deux électrodes de décharge (50) et/ou les transmettre acoustiquement et/ou visuellement à un utilisateur.

15. Système (100) selon la revendication 14, le système (100) comprenant en outre soit un appareil de mesure d'impédance, qui est relié de manière communicative avec l'unité de surveillance et est conçu pour effectuer une mesure d'impédance, soit alternativement, dans lequel le système IOM (30) est conçu pour exécuter la mesure d'impédance, pour la mesure d'impédance, des résistances complexes de tissus entourant la pointe de l'unité de canule (5) étant mesurées à différentes fréquences et le système étant conçu, en se basant sur les impédances mesurées, pourndéterminer le type de tissu et le transmettre à l'utilisateur.
